# EUROPEAN PATENT APPLICATION

(11) **EP 1 108 725 A1**
(43) Date of publication of application: **20.06.2001**
(21) Application number: 99939619.5
(22) Date of filing: 26.08.1999
(51) Int. Cl.: C07K 14/47, C12N 15/12, C12N 1/21, C12N 5/10, C12P 21/02, C12Q 1/68, C07K 16/18, G01N 33/15, G01N 33/53, A61K 38/16, A61K 48/00, A61K 39/395

(54) **NOVEL POLYPEPTIDE**

(30) Priority: 27.08.1998 JP 24124898
(71) Applicant: KYOWA HAKKO KOGYO CO., LTD., Chiyoda-ku, Tokyo 100-8185 (JP)
(72) Inventor: MIYAJI, Hiromasa, Sunto-gun, Shizuoka 411-0945 (JP); MIMURA, Hideki, Sunto-gun, Shizuoka 411-0945 (JP); HARUOKA,Motoko, Sunto-gun, Shizuoka 411-0943 (JP); NAKAGAWA, Satoshi, Machida-shi, Tokyo 194-0021 (JP)
(74) Representative: VOSSIUS & PARTNER
(86) International application number: JP9904602
(87) International publication number: WO0012550

(57) **Abstract**

This invention relates to a novel transporter polypeptide, a DNA encoding this polypeptide, a vector containing this DNA, a transformant harboring this vector and a method for producing the transporter polypeptide.
The present invention also relates to an antibody specifically reacting with the polypeptide, a microorganism, an animal cell or an animal producing this antibody, a method for searching a compound having a ligand activity using the polypeptide, a part thereof or a microorganism, an animal cell or the like with the expression of the same and a method for searching a compound regulating the expression of the gene of the polypeptide using cells.

SEQUENCE LISTING

<110> KYOWA HAKKO KOGYO CO., LTD.,

<120> NOVEL POLYPEPTIDE

<130> 11152WO1

<150> JP H10-241248
<151> 1998-08-27
<160> 8

<170> PatentIn Ver. 2.0

<210> 1
<211> 475
<212> PRT
<213> Homo sapiens

<400> 1

<210> 2
<211> 2240
<212> DNA
<213> Homo sapiens

<220>
<221> CDS
<222> (24)..(1451)

<400> 2
cggcggcgtg gcgcagcggc gac atg gcc gtt gtc tca gag gac gac ttt cag 53
cac agt tca aac tcc acc tac gga acc aca agc agc agt ctc cga gct    101
gac cag gag gca ctg ctt gag aag ctg ctg gac cgc ccg ccc cct ggc    149
ctg cag agg ccc gag gac cgc ttc tgt ggc aca tac atc atc ttc ttc 197
agc ctg ggc att ggc agt cta ctg cca tgg aac ttc ttt atc act gcc 245
aag gag tac tgg atg ttc aaa ctc cgc aac tcc tcc agc cca gcc acc 293
ggg gag gac cct gag ggc tca gac atc ctg aac tac ttt gag agc tac 341
ctt gcc gtt gcc tcc acc gtg ccc tcc atg ctg tgc ctg gtg gcc aac 389
ttc ctg ctt gtc aac agg gtt gca gtc cac atc cgt gtc ctg gcc tca 437
ctg acg gtc atc ctg gcc atc ttc atg gtg ata act gca ctg gtg aag 485
gtg gac act ttc tcc tgg acc cgt ggc ttt ttt gcg gtc acc att gtc 533
tgc atg gtg atc ctc agc ggt gcc tcc act gtc ttc agc agc agc atc 581
tac ggc atg acc ggc tcc ttt cct atg agg aac tcc cag gca ctg ata 629
tca gga gga gcc atg ggc ggg acg gtc agc gcc gtg gcc tca ttg gtg 677
gac ttg gct gca tcc agt gat gtg agg aac agc gcc ctg gcc ttc ttc 725
ctg acg gcc acc atc ttc ctc gtg ctc tgc atg gga ctc tac ctg ctg 773
ctg tcc agg ctg gag tat gcc agg tac tac atg agg cct gtt ctt gcg 821
gcc cat gtg ttt tct ggt gaa gag gag ctt ccc cag gac tcc ctc agt 869
gcc cct tcg gtg gcc tcc aga ttc att gat tcc cac aca ccc cct ctc 917
cgc ccc atc ctg aag aag acg gcc agc ctg ggc ttc tgt gtc acc tac 965
gtc ttc ttc atc acc agc ctc atc tac ccc gcc gtc tgc acc aac atc 1013
gag tcc ctc aac aag ggc tcg ggc tca ctg tgg acc acc aag ttt ttc 1061
atc ccc ctc act acc ttc ctc ctg tac aac ttt gct gac cta tgt ggc 1109
cgg cag ctc acc gcc tgg atc cag gtg cca ggg ccc aat agc aag gcg 1157
ctc cca ggg ttc gtg ctc ctc cgg acc tgc ctc atc ccc ctc ttc gtg 1205
ctc tgt aac tac cag ccc cgc gtc cac ctg aag act gtg gtc ttc cag 1253
tcc gat gtg tac ccc gca ctc ctc agc tcc ctg ctg ggg ctc agc aac 1301
ggc tac ctc agc acc ctg gcc ctc ctc tac ggg cct aag att gtg ccc 1349
agg gag ctg gct gag gcc acg gga gtg gtg atg tcc ttt tat gtg tgc 1397
ttg ggc tta aca ctg ggc tca gcc tgc tct acc ctc ctg gtg cac ctc 1445
atc tag aagggaggac acaaggacat tggtgcttca gagcctttga agatgagaag 1501
agagtgcagg agggctgggg gccatggagg aaaggcctaa agtttcactt ggggacagag 1561
agcagagcac actcgggcct catccctccc aagatgccag tgagccacgt ccatgcccat 1621
tccgtgcaag gcagatattc cagtcatatt aacagaacac tcctgagaca gttgaagaag 1681
aaatagcaca aatcaggggt actcccttca cagctgatgg ttaacattcc accttctttc 1741
tagcccttca aagatgctgc cagtgttcgc cctagagtta ttacaaagcc agtgccaaaa 1801
cccagccatg ggctctttgc aacctcccag ctgcgctcat tccagctgac agcgagatgc 1861
aagcaaatgc tcagctctcc ttaccctgaa ggggtctccc tggaatggaa gtcccctggc 1921
atggtcagtc ctcaggccca agactcaagt gtgcacagac ccctgtgttc tgtgggtgaa 1981
caactgccca ctaaccagac tggaaaaccc agaaagatgg gccttccatg aatgcttcat 2041
tccagaggga ccagagggcc tccctgtgca agggatcaag catgtctggc ctgggttttc 2101
aaaaaaagag ggatcctcat gacctggtgg tctatggcct gggtcaagat gagggtcttt 2161
cagtgttcct gtttacaaca tgtcaaagcc attggttcaa gggcgtaata aatacttgcg 2221
tattcaaaaa aaaaaaaaa 2240

<210> 3
<211> 20
<212> DNA
<213> Artificial Sequence

<220>
<223> Description of Artificial Sequence: synthetic DNA

<400> 3
actttgctga cctacgtggc 20

<210> 4
<211> 20
<212> DNA
<213> Artificial Sequence

<220>
<223> Description of Artificial Sequence: synthetic DNA

<400> 4
tacgcccttg aaccaatggc 20

<210> 5
<211> 475

<212> PRT

<213> Rat

<400> 5

Met Ala Phe Ala Ser Glu Asp Ile Ala Tyr His Ser Ser Asn Ala Val
1               5                   10                  15

Tyr Arg Val Pro Ser Asn Arg His Glu Ala Asp Gln Glu Ala Leu Leu
            20                  25                  30

Gly Lys Pro Leu Asp Tyr Pro Ala Pro Gly Leu Gln Arg Pro Glu Asp
        35                  40                  45

Arg Phe Asn Gly Ala Tyr Ile Ile Phe Phe Cys Leu Gly Ile Gly Gly
    50                  55                  60

Leu Leu Pro Trp Asn Phe Phe Val Thr Ala Lys Glu Tyr Trp Ala Phe
65                  70                  75                  80

Lys Leu Arg Asn Cys Ser Ser Pro Ala Ser Gly Lys Asp Pro Glu Asp
                85                  90                  95

Ala Asp Ile Leu Asn Tyr Phe Glu Ser Tyr Leu Ala Val Ala Ser Thr
            100                 105                 110

Val Pro Ser Leu Leu Phe Leu Val Ala Asn Phe Leu Leu Val Asn Arg
        115                 120                 125

Ile Arg Val His Val Arg Val Leu Ala Ser Leu Ser Val Ser Leu Ala
    130                 135                 140

Ile Phe Val Val Met Ala Val Leu Val Arg Val Asp Thr Ser Ser Trp
145                 150                 155                 160

Thr Arg Gly Phe Phe Ser Ile Ala Met Ala Cys Met Ala Ile Ile Ser
                165                 170                 175

Ser Ser Ser Thr Ile Phe Asn Ser Ser Val Tyr Gly Leu Thr Gly Ser
            180                 185                 190

Phe Pro Met Arg Asn Ala Gln Ala Leu Ile Ser Gly Gly Ala Met Gly
        195                 200                 205

Gly Thr Val Ser Ala Val Ala Ser Leu Val Asp Leu Ala Ala Ser Ser
    210                 215                 220

Asp Val Arg Asp Ser Ala Leu Ala Phe Phe Leu Thr Ala Ala Val Phe
225                 230                 235                 240

Leu Gly Leu Cys Val Gly Leu Tyr Leu Leu Leu Pro Gln Leu Glu Tyr
                245                 250                 255

Ala Arg Tyr Tyr Met Arg Pro Val Val Pro Ile His Val Phe Ser Ser
            260                 265                 270

Glu Asp Ser Pro Pro Arg Asp Ala Pro Ser Thr Ser Ser Val Ala Pro
        275                 280                 285

Ala Ser Arg Ala Val His Thr Pro Pro Leu Gly Pro Ile Leu Lys Lys
    290                 295                 300

Thr Ala Gly Leu Gly Phe Cys Ala Val Phe Leu Tyr Phe Ile Thr Ala
305                 310                 315                 320

Leu Ile Phe Pro Ala Ile Ser Thr Asn Ile Gln Pro Met His Lys Gly
                325                 330                 335

Thr Gly Ser Pro Trp Thr Ser Lys Phe Tyr Val Pro Leu Thr Val Phe
            340                 345                 350

Leu Leu Phe Asn Phe Ala Asp Leu Cys Gly Arg Gln Val Thr Ala Trp
        355                 360                 365

Ile Gln Val Pro Gly Pro Arg Ser Lys Leu Leu Pro Ile Leu Ala Val
    370                 375                 380

Ser Arg Val Cys Leu Val Pro Leu Phe Leu Leu Cys Asn Tyr Gln Pro
385                 390                 395                 400

Arg Ser His Leu Thr Leu Val Leu Phe Gln Ser Asp Ile Tyr Pro Ile
                405                 410                 415

Leu Phe Thr Cys Leu Leu Gly Leu Ser Asn Gly Tyr Leu Ser Thr Leu
            420                 425                 430

Val Leu Met Tyr Gly Pro Lys Ile Val Pro Arg Glu Leu Ala Glu Ala
        435                 440                 445

Thr Ser Val Val Met Leu Phe Tyr Met Ser Leu Gly Leu Met Leu Gly
    450                 455                 460

Ser Ala Cys Ala Ala Leu Leu Glu His Phe Ile
465                 470                 475

<210> 6

<211> 1734

<212> DNA

<213> Rat

<220>
<221> CDS

<222> (12)..(1436)

<223> Description of Artificial Sequence: synthetic DNA

<400> 6

gcggcagcga c atg gcc ttt gcc tct gag gac att gca tac cac agc tca      50
             Met Ala Phe Ala Ser Glu Asp Ile Ala Tyr His Ser Ser
             1               5                   10

aat gct gtc tac aga gtc cca agc aac cgt cat gaa gct gac cag gaa      98
Asn Ala Val Tyr Arg Val Pro Ser Asn Arg His Glu Ala Asp Gln Glu
    15                  20                  25

gcc cta ctg gga aaa cca cta gac tac cca gcc cca ggc ctg cag agg      146
Ala Leu Leu Gly Lys Pro Leu Asp Tyr Pro Ala Pro Gly Leu Gln Arg
30                  35                  40                  45

cca gag gac cgc ttc aat ggt gcc tat atc atc ttc ttc tgc ctg gga      194
Pro Glu Asp Arg Phe Asn Gly Ala Tyr Ile Ile Phe Phe Cys Leu Gly
                50                  55                  60

att ggc ggc cta cta ccc tgg aac ttt ttt gtc act gcc aaa gag tac      242
Ile Gly Gly Leu Leu Pro Trp Asn Phe Phe Val Thr Ala Lys Glu Tyr
            65                  70                  75

tgg gca ttt aaa ctc cga aac tgc tcc agc cca gcc tcc ggg aag gac      290
Trp Ala Phe Lys Leu Arg Asn Cys Ser Ser Pro Ala Ser Gly Lys Asp

80                    85                    90

cca gag gat gca gac atc ctg aac tac ttt gag agc tac ctg gcg gtt     338
Pro Glu Asp Ala Asp Ile Leu Asn Tyr Phe Glu Ser Tyr Leu Ala Val
    95                  100                 105

gct tcc act gtg cct tcc ctg ctg ttt ctg gtg gct aac ttc ctg ctt     386
Ala Ser Thr Val Pro Ser Leu Leu Phe Leu Val Ala Asn Phe Leu Leu
110                 115                 120                 125

gtc aac agg atc cgg gtg cat gtc cga gtt ctg gcc tca ctg tcc gtc     434
Val Asn Arg Ile Arg Val His Val Arg Val Leu Ala Ser Leu Ser Val
                130                 135                 140

tcc ctg gcc atc ttt gtg gtt atg gcc gtg ctg gtg agg gtg gac act     482
Ser Leu Ala Ile Phe Val Val Met Ala Val Leu Val Arg Val Asp Thr
            145                 150                 155

tct tcc tgg acc cgg ggc ttc ttc agc atc gcc atg gcg tgc atg gcc     530
Ser Ser Trp Thr Arg Gly Phe Phe Ser Ile Ala Met Ala Cys Met Ala
        160                 165                 170

atc atc agc agc tcc tcc acc atc ttc aat agc agc gtg tat ggc ctg     578
Ile Ile Ser Ser Ser Ser Thr Ile Phe Asn Ser Ser Val Tyr Gly Leu
    175                 180                 185

acg ggc tcg ttc ccc atg agg aat gcc cag gca ctg ata tca gga gga     626
Thr Gly Ser Phe Pro Met Arg Asn Ala Gln Ala Leu Ile Ser Gly Gly
190                 195                 200                 205

gcc atg gga ggg aca gtc agc gcc gtg gcc tcc ctg gtg gac ctg gca     674
Ala Met Gly Gly Thr Val Ser Ala Val Ala Ser Leu Val Asp Leu Ala
                210                 215                 220

gca tcc agt gac gtg cga gac agt gcg ctg gcc ttc ttc ctc aca gca     722
Ala Ser Ser Asp Val Arg Asp Ser Ala Leu Ala Phe Phe Leu Thr Ala
            225                 230                 235

gca gtc ttc ctt ggg ctc tgt gtg ggg ctc tac cta ctg ctg ccc caa 770
Ala Val Phe Leu Gly Leu Cys Val Gly Leu Tyr Leu Leu Leu Pro Gln
        240                 245                 250

ctg gag tat gcc agg tac tac atg agg ccg gtt gtc cca atc cac gtg 818
Leu Glu Tyr Ala Arg Tyr Tyr Met Arg Pro Val Val Pro Ile His Val
    255                 260                 265

ttt tct agt gaa gac agc cca ccc cgg gat gct ccc agc acc tcc tcc 866
Phe Ser Ser Glu Asp Ser Pro Pro Arg Asp Ala Pro Ser Thr Ser Ser
270                 275                 280                 285

gtg gcc cct gca tcc aga gca gtg cac acg cca ccc ctc gga ccc atc 914
Val Ala Pro Ala Ser Arg Ala Val His Thr Pro Pro Leu Gly Pro Ile
                290                 295                 300

ctg aag aag aca gct ggc ctg ggg ttc tgc gcc gtt ttc ctc tac ttc 962
Leu Lys Lys Thr Ala Gly Leu Gly Phe Cys Ala Val Phe Leu Tyr Phe
            305                 310                 315

atc acc gcc ctt atc ttc ccc gct atc tcc acc aac atc cag ccc atg 1010
Ile Thr Ala Leu Ile Phe Pro Ala Ile Ser Thr Asn Ile Gln Pro Met
        320                 325                 330

cac aag ggc acc ggc tct cca tgg acc tcc aag ttc tat gtg ccc ctc 1058
His Lys Gly Thr Gly Ser Pro Trp Thr Ser Lys Phe Tyr Val Pro Leu
    335                 340                 345

acc gtc ttc ctc ctt ttc aac ttt gct gac ctc tgc ggc cga cag gtc 1106
Thr Val Phe Leu Leu Phe Asn Phe Ala Asp Leu Cys Gly Arg Gln Val
350                 355                 360                 365

aca gcc tgg atc cag gtg cca ggt cct agg agc aag ctg ctc ccc ata 1154
Thr Ala Trp Ile Gln Val Pro Gly Pro Arg Ser Lys Leu Leu Pro Ile
                370                 375                 380

ctg gca gtc tct cgc gtc tgc ctc gtg cct ctc ttc ctg ctc tgt aac 1202
Leu Ala Val Ser Arg Val Cys Leu Val Pro Leu Phe Leu Leu Cys Asn

385                 390                 395

tac cag cca cgc tca cac ctg act ctg gtg ctt ttc cag tct gac atc     1250
Tyr Gln Pro Arg Ser His Leu Thr Leu Val Leu Phe Gln Ser Asp Ile
        400                 405                 410

tac cct ata ctc ttc acc tgc ctc ttg ggg ctc agt aat ggc tac ctc     1298
Tyr Pro Ile Leu Phe Thr Cys Leu Leu Gly Leu Ser Asn Gly Tyr Leu
    415                 420                 425

agc acg ctg gtg ctc atg tat ggg ccc aag att gtg ccc cgg gag ctg     1346
Ser Thr Leu Val Leu Met Tyr Gly Pro Lys Ile Val Pro Arg Glu Leu
430                 435                 440                 445

gct gag gcc acc agt gtg gtg atg ctg ttc tac atg tca ctg ggt ttg     1394
Ala Glu Ala Thr Ser Val Val Met Leu Phe Tyr Met Ser Leu Gly Leu
                450                 455                 460

atg ctg ggc tca gcc tgt gcg gcc ttg ctt gag cac ttt atc taggagggg  1445
Met Leu Gly Ser Ala Cys Ala Ala Leu Leu Glu His Phe Ile
            465                 470                 475

cggcaaggat gtgggttctg tgtgagtgag tgtggtttgg gtccctggga cctggacagg  1505
gtgagccgag gtctcatggc gttaagcaag gggttggtgt tttgcttgat gtacagcaga  1565
gcccactcag actgtctttc tctcagacac atccttgcac atcttgttca aggagacatt  1625
ccagacacag cccagcatgg tggctcacac ctgtaatccc agcattcaag aggctggagc  1685
tagaggactg ctgtgagttc aaggccagcc tggctccata gtaaaaccc              1734

<210> 7

<211> 14

<212> DNA

<213> Artificial Sequence

<220>

<223> Description of Artificial Sequence: synthetic DNA

<400> 7

arrtanccrt tact                                                    14

<210> 8
<211> 17
<212> DNA
<213> Artificial Sequence

<220>
<223> Description of Artificial Sequence: synthetic DNA

<400> 8
ytnccntgga acttttt                                                 17

## Description

### Technical Field

This invention relates to a novel transporter polypeptide, a DNA encoding this polypeptide, a vector containing this DNA, a transformant harboring this vector and a method for producing the transporter polypeptide. The present invention also relates to an antibody specifically reacting with the polypeptide, a microorganism, an animal cell or an animal producing this antibody, a method for searching a compound having a ligand activity using the polypeptide, a part thereof or a microorganism, an animal cell or the like with the expression of the same and a method for searching a compound regulating the expression of the gene of the polypeptide using cells.

### Background Art

It is known that physiological incorporation of nucleosides such as adenosine, cytidine, guanosine, inosine, thymidine, uridine and the like into mammal cells is carried out by the mechanism of Na⁺-dependence and Na⁺-independence {Drug Transport in Antimicrobial and Anticancer Chemotherapy, pp. 403 - 451, Marcel Decker, New York (1995), *Biochim*. *Biophys*. *Acta.,* 1286, 153 (1996)}.

Such a mechanism is essential in the synthesis of nucleotides and nucleic acids by the salvage pathway in de *novo* pathway-deficient hemopoietic cells and the like and is also concerned in the intracellular incorporation of many cytotoxic nucleoside derivatives which are used in antitumor and antiviral treatment of leukemia, AIDS and the like {*Nucleosides Nucleotides*, 11, 903 (1992), *J. Antimicrobiol. Chemother.,* 32, Suppl. A. 133 (1993)}.

In addition, it has been reported that the nucleoside transport process also plays an important role in various physiological activities mediated by adenosine, illustratively in coronary artery vasodilatation, renal vasoconstriction, neurotransmission, platelet aggregation, lipolysis and the like {*Prog*. *Cardiovasc*. *Dis*., 32, 73 (1989), Purines in Cellular Signaling: Targets for New Drugs, Springer-Verlag, New York (1990), Adenosine and Adenine Nucleotides: From Molecular Biology to Integrative Physiology, Kluwer Academic Publishers, Boston (1995)}.

The Na⁺-dependent nucleoside transporter (concentrative nucleoside transporter) is expressed locally in functionally specialized cells such as epithelia of the intestines and kidney, choroidal plexus, the liver, macrophage, spleen cells or leukemia cells and incorporates nucleosides into cells, which is driven by the Na⁺ concentration gradient across the plasma membrane {Drug Transport in Antimicrobial and Anticancer Chemotherapy, pp. 403 - 451, Marcel Decker, New York (1995), *Biochim*. *Biophys*. *Acta.*, 1286, 153 (1996)}.

On the other hand, the Na⁺-independent nucleoside transporter is generally expressed in various cells and tissues, concerning in both outflow and inflow of nucleosides via plasma membrane, and is therefore called equilibrative nucleoside transporter (ENT).

Based on its sensitivity to an inhibitor nitrobenzylthioinosine (NBMPR; 6-{(4-nitrobenzyl)thio}-9-β-D-ribofuranosylpurine), ENT is classified into two types, namely an equilibrative sensitive (es) transporter which shows high affinity for NBMPR (Kd = 0.1 to 10 nM) and equilibrative insensitive (ei) transporter which is not inhibited by NBMPR at a low concentration (nM order) but inhibited at a high concentration (µM order) {Drug Transport in Antimicrobial and Anticancer Chemotherapy, pp. 403 - 451, Marcel Decker, New York (1995), *Biochim*. *Biophys*. *Acta*., 1286, 153 (1996)}.

It is known that the es type transporter is a pharmacological target of coronary artery vasodilator agents such as dipyridamole, dilazep and the like *{Prog. Cardiovasc*. *Dis.,* 32, 73 (1989), Purines in Cellular Signaling: Targets for New Drugs, Springer-Verlag, New York (1990)}.

Regarding ENT, cloning of human and rat es type transporter cDNA (hENT1 and rENT1, respectively) and human and rat ei type transporter cDNA (hENT2 and rENT2, respectively) has so far been reported {*Nature Medicine,* 3, 89 (1997), *J. Biol*. *Chem.,* 272, 28423 (1997), *J*. *Biol*. *Chem*., 273, 5288 (1998), *Biochemical J.,* 328, *739* (1997)}.

These equilibrative transporters are considered to be eleven-transmembrane-spanning glycoproteins, and mutual homology is recognized (about 60% of identity between hENT1 and hENT2 at amino acid level and about 50% of identity between rENT1 and rENT2 at amino acid level).

Regarding hENT1, significant homology with concentrative transporter cNT1 {*J. Biol*. *Chem*., 269, 17757 (1994)} or with bacterial nucleoside transporters nupC {*Mol. Microbiol*., 11, 1159 (1994)} and nupG {*Eur*. *J. Biochem*., 168, 385 (1987)} has not been found.

On the other hand, 23% and 21% identity was observed at amino acid level with ZK809.4 and F16H11.3, respectively, which had been found in a genome project of a roundworm (*Caenorhabditis elegans*). Also, 20% identity was found with FUN26 of a yeast (*Saccharomyces cerevisiae*). Although functions of these proteins are not clear, this indicates a possibility of nucleoside transporters existing in corresponding organisms {*Nature Medicine*, 3, 89 (1997)}.

Since heterogeneity is observed in ENT based on cells and tissues, a possibility of the presence of other ENT isoforms has been suggested as well {*Biochim*. *Biophys. Acta*., 1286, 153 (1996)}.

### Disclosure of the Invention

The present invention is to provide a novel transporter polypeptide which transports nucleoside molecules and the like into cells or discharge them from cells, a DNA which encodes the transporter polypeptide and a preventive agent or a therapeutic agent for ischemic heart disease, cerebral disorder at the time of stroke, immunoreaction accompanied by organ transplantation, malignant tumor, glomerular nephritis, pancreatitis, hypertension or the like, using an antibody which recognizes the polypeptide.

Based on the gene sequence information of hENT1, the inventors of the present invention have analyzed the EST (Expressed Sequence Tag) registered in a gene sequence database of random human cDNA sequences, Genbank, using a Frame Search (manufactured by Compugen, Israel) homology searching software, and found partial sequences having homology with hENT1 (R07250 and AA608799). Using these EST sequences, cDNA of a novel transporter polypeptide was obtained and its nucleotide sequence was analyzed. Moreover, a rat counterpart was also obtained, thus resulting in the accomplishment of the present invention.

Accordingly, the present invention relates to the following inventions of (1) to (45).
(1) A polypeptide which comprises the amino acid sequence described in SEQ ID NO:1 or 5.
(2) A polypeptide which comprises an amino acid sequence of the amino acid sequence described in SEQ ID NO:1 or 5 wherein one or several amino acids are deleted, substituted or added and also has a nucleoside transporting activity.
   The just described deletion, substitution or addition of amino acids can be carried out by site-directed mutagenesis which is a known technique prior to the application, and the term "one or several amino acids" means the number of amino acids which can be deleted, substituted or added by the site-directed mutagenesis.
   Such a polypeptide comprising an amino acid sequence wherein one or several amino acids are deleted, substituted or added and also having the activity to transport a nucleoside can be prepared in accordance with the methods described in Molecular Cloning, A Laboratory Manual, Second Edition, Cold Spring Harbor Laboratory Press (1989) (to be referred to as "Molecular Cloning, Second Edition" hereinafter), Current Protocols in Molecular Biology, Supplement 1 - 38, John Wiley & Sons (1987 - 1997) (to be referred to as "Current Protocols in Molecular Biology" hereinafter), *Nucleic Acids Research,* 10, 6487 (1982), *Proc*. *Natl*. *Acad*. *Sci. USA,* 79, 6409 (1982), *Gene,* 34, 315 (1985), *Nucleic Acids Research,* 13*,* 4431 (1985), *Proc. Natl*. *Acad*. *Sci. USA,* 82, 488 (1985), *Proc*. *Natl*. *Acad. Sci*. *USA*, 81, 5662 (1984), *Science,* 224, 1431 (1984), PCT WO 85/00817 (1985), *Nature,* 316, 601 (1985) and the like.
   In this connection, the polypeptide of the present invention does not encompass known polypeptides.
(3) A DNA which encodes the aforementioned polypeptide of item (1) or (2).
(4) A DNA which has the nucleotide sequence described in SEQ ID NO:2 or 6.
(5) A DNA which hybridizes with the DNA of aforementioned item (3) or (4) under stringent conditions and also encodes a polypeptide having a nucleoside transporting activity.
   The just described term "DNA which hybridizes under stringent conditions and also encodes a polypeptide having a nucleoside transporting activity" means a DNA which is obtained by the colony hybridization method, the plaque hybridization method, the Southern blot hybridization method or the like using the aforementioned DNA of item (3) or (4) as a probe, and its illustrative example is a DNA which can be identified by carrying out hybridization at 65°C in the presence of 0.7 to 1.0 M NaCl using a filter on which colony- or plaque-derived DNA fragments are fixed and then washing the filter at 65°C using 0.1 to 2 x SSC (saline-sodium citrate) solution (composition of 1 x SSC solution is composed of 150 mM sodium chloride and 15 mM sodium citrate).
   The hybridization can be carried out in accordance with the method described in a textbook such as Molecular Cloning Second Edition, Current Protocols in Molecular Biology or DNA Cloning 1: Core Techniques, A Practical Approach, Second Edition, Oxford University Press (1995).
   Illustrative examples of the hybridizable DNA include a DNA having at least 80% or more of homology, preferably a DNA having 95% or more of homology, with the nucleotide sequence represented by the SEQ ID NO:2 or 6.
   In addition, the DNA of the present invention does not encompass known DNAs.
(6) A recombinant DNA which is obtained by integrating the DNA described in any one of the aforementioned items (3) to (5) into a vector.
(7) The recombinant DNA according to the item (6), wherein the recombinant DNA is a plasmid p46-1 or p3-2.
(8) A transformant which harbours the recombinant DNA of the item (6) or (7).
(9) The transformant according to the above item (8), wherein the transformant is a transformant selected from a microorganism, an animal cell, a plant cell and an insect cell.
(10) The transformant according to the above item (9), wherein the microorganism is a microorganism belonging to the genus *Escherichia.*
(11) The transformant according to the above item (10), wherein the microorganism belonging to the genus *Escherichia* is *Escherichia coli* JM109/p46-1 (FERM BP-6462) or *Escherichia coli* JM109/p3-2 (FERM BP-6830).
(12) A method for producing the polypeptide of the aforementioned item (1) or (2), which comprises culturing the transformant described in any one of the aforementioned items (8) to (11) in a medium to form and accumulate the polypeptide of item (1) or (2) in the culture, and subsequently recovering the polypeptide from the culture.
(13) An oligonucleotide which is selected from an oligonucleotides comprising a sequence identical to continuous 5 to 60 bases in a nucleotide sequence of the DNA described in any one of the aforementioned items (3) to (5), an oligonucleotide having a sequence complementary to the above oligonucleotide and oligonucleotide derivatives of these oligonucleotides.
(14) The oligonucleotide according to the above item (13), which is selected from oligonucleotide derivatives in which phosphodiester linkage in the oligonucleotide is substituted by phosphorothioate bond, an oligonucleotide derivative in which phosphodiester linkage in the oligonucleotide is substituted by N3'-P5' phosphoamidate bond, an oligonucleotide derivative in which ribose and phosphodiester linkage in the oligonucleotide are substituted by peptide-nucleic acid linkage, an oligonucleotide derivative in which uracil in the oligonucleotide is substituted by C-5 propynyluracil, an oligonucleotide derivative in which uracil in the oligonucleotide is substituted by C-5 thiazoleuracil, an oligonucleotide derivative in which cytosine in the oligonucleotide is substituted by C-5 propynylcytosine, an oligonucleotide derivative in which cytosine in the oligonucleotide is substituted by phenoxazine-modified cytosine, an oligonucleotide derivative in which ribose in the DNA is substituted by 2'-O-propylribose and an oligonucleotide derivative in which ribose in the oligonucleotide is substituted by 2'-methoxyethoxyribose.
(15) A method for detecting mRNA encoding the aforementioned polypeptide of item (1) or (2) using the oligonucleotide of the above item (13) or (14).
(16) A method for inhibiting expression of the aforementioned polypeptide of item (1) or (2) using the oligonucleotide of the above item (13) or (14).
(17) An antibody which recognizes the aforementioned polypeptide of item (1) or (2).
(18) An immunological detection method of the aforementioned polypeptide of item (1) or (2) or an immunohistological staining method, which comprises using the above antibody of item (17).
(19) An immunohistological staining agent which contains the above antibody of item (17).
(20) A method for screening a compound capable of changing the activity to transport a nucleoside of the aforementioned polypeptide of item (1) or (2), which comprises contacting the polypeptide with a test sample.
(21) A compound obtainable by the method of the above item (20).
(22) A method for screening a compound capable of changing expression of a gene encoding the aforementioned polypeptide of item (1) or (2), which comprises contacting a cell expressing the polypeptide with a test sample.
(23) The screening method according to the above item (22), wherein detection of changes in the expression of the gene encoding the aforementioned polypeptide of item (1) or (2) is carried out by detecting mRNA encoding the polypeptide using the aforementioned method of item (15).
(24) The screening method according to the above item (22), wherein detection of changes in the expression of the gene encoding the aforementioned polypeptide of item (1) or (2) is carried out by detecting the polypeptide using the aforementioned method of item (18).
(25) A compound obtainable by any one of the methods of the above items (22) to (24).
(26) A preventive agent or a therapeutic agent for ischemic heart disease, cerebral disorder at the time of stroke, immune response accompanied by organ transplantation, malignant tumor, nephritis, pancreatitis or hypertension in a mammal, which comprises the aforementioned polypeptide of item (1) or (2).
(27) An agent for increasing activity of an antiviral agent or a malignant tumor treating agent for a mammal, which comprises the aforementioned polypeptide of item (1) or (2).
(28) An analgesic or an antiplatelet agent for a mammal, which comprises the aforementioned polypeptide of item (1) or (2).
(29) An agent for reducing side effects at the time of chemotherapy of a mammal, which comprises the aforementioned polypeptide of item (1) or (2).
(30) A preventive agent or a therapeutic agent for ischemic heart disease, cerebral disorder at the time of stroke, immune response accompanied by organ transplantation, malignant tumor, nephritis, pancreatitis or hypertension in a mammal, which comprises the aforementioned oligonucleotide of item (13) or (14).
(31) An agent for increasing activity of an antiviral agent or a malignant tumor treating agent for a mammal, which comprises the aforementioned oligonucleotide of item (13) or (14).
(32) An analgesic or an antiplatelet agent for a mammal, which contains the aforementioned oligonucleotide of item (13) or (14).
(33) An agent for reducing side effects at the time of chemotherapy of a mammal, which comprises the aforementioned oligonucleotide of item (13) or (14).
(34) A preventive agent or a therapeutic agent for ischemic heart disease, cerebral disorder at the time of stroke, immune response accompanied by organ transplantation, malignant tumor, nephritis, pancreatitis or hypertension in a mammal, which comprises the aforementioned antibody of item (17).
(35) An agent for increasing activity of an antiviral agent or a malignant tumor treating agent for a mammal, which comprises the aforementioned antibody of item (17).
(36) An analgesic or an antiplatelet agent for a mammal, which comprises the aforementioned antibody of item (17).
(37) An agent for reducing side effects at the time of chemotherapy of a mammal, which comprises the aforementioned antibody of item (17).
(38) The preventive agent or a therapeutic agent for ischemic heart disease, cerebral disorder at the time of stroke, immune response accompanied by organ transplantation, malignant tumor, nephritis, pancreatitis or hypertension according to any one of the aforementioned items (26), (30) and (34), wherein the mammal is human.
(39) The agent for increasing activity of an antiviral agent or a malignant tumor treating agent according to any one of the aforementioned items (27), (31) and (35), wherein the mammal is human.
(40) The analgesic or antiplatelet agent according to any one of the aforementioned items (28), (32) and (36), wherein the mammal is human.
(41) The agent for reducing side effects at the time of chemotherapy according to any one of the aforementioned items (29), (33) and (37), wherein the mammal is human.
(42) A promoter DNA which controls transcription of a gene encoding the aforementioned polypeptide of item (1) or (2).
(43) A method for screening a compound capable of changing efficiency of transcription by the promoter of the above item (42), which comprises contacting a transformant harboring a plasmid containing the promoter DNA and a reporter gene connected to the downstream of the promoter DNA with a test sample and measuring the content of translation product of the reporter gene.
(44) The screening method according to the above item (43), wherein the reporter gene is a gene selected from a chloramphenicol acetyltransferase gene, a β-galactosidase gene, a luciferase gene and a green fluorescent protein gene.
(45) A compound obtainable by the method of the above item (43) or (44).

In the following, the present invention is described in detail.

### {1} Acquisition of the DNA of the present invention and preparation of oligonucleotide

A gene having homology with hENT1 {*Nature Medicine,* 3, 89 (1997)} is screened from gene databases or protein databases using a program which uses the Blast, Smith-Waterman method or the like or the Frame Search (manufactured by Compugen, Israel) homology searching software.

As the database, public databases such as Genbank, Swiss-Plot or the like can be used.

When the thus obtained gene having homology with hENT1 is a nucleotide sequence which is only a part of nucleotide sequence of the gene such as the case of EST (Expressed Sequence Tag) or an hENT1 homologous gene of mammal other than human such as rat or the like, full-length of its cDNA can be obtained in the following manner, and the DNA of the present invention can be obtained from the cDNA.

### (1) Preparation of cDNA library

In order to prepare a cDNA library, total RNA or mRNA is prepared from an appropriate cell or tissue.

Examples of the method for preparing total RNA include the guanidine thiocyanate-cesium trifluoroacetate method {*Methods in Enzymology,* 154, 3 (1987)} and the acid guanidium thiocyanate phenol chloroform (AGPC) method {*Analytical Biochemistry*, 162, 156 (1987), *Jikken Igaku* (Experimental Medicine), 9, 1937 (1991)}.

Examples of the method for preparing mRNA as poly(A)⁺ RNA from the total RNA include the oligo(dT) immobilized cellulose column method (Molecular Cloning, Second Edition) and the oligo-dT latex-aided method {*Saibo Kogaku* (Cell Technology), Supplement 8 "New Cell Engineering Experimentation Protocol", published by Shujun-sha, pp. 48 - 52 (1993), *Nucleic Acids Res.,* Symposium Series, 19, 61 (1988)}.

Also, mRNA can be prepared directly from a tissue or cell using Fast Track mRNA Isolation Kit (manufactured by Invitrogen), Quick Prep mRNA Purification Kit (manufactured by Pharmacia) or the like.

As the appropriate cell or tissue, it is desirable to examine the cDNA library which contained the EST or the like, found in the database, and to use the cell or tissue, or a cell strain or the like derived from the tissue, for constructing the library.

Using the thus obtained total RNA or mRNA, a cDNA library is prepared by a conventional method.

Examples of the cDNA library preparation method include the method described in Molecular Cloning, Second Edition, Current Protocols in Molecular Biology, DNA Cloning 1: Core Techniques, A Practical Approach, Second Edition, Oxford University Press (1995) and a method which uses a commercially available kit such as Superscript Plasmid System for cDNA Synthesis and Plasmid Cloning (manufactured by Gibco BRL) or ZAP-cDNA Synthesis Kit (manufactured by Stratagene).

As the cloning vector for preparing a cDNA library, any one of phage vectors, plasmid vectors and the like can be used as long as it can be autonomously replicated in *Escherichia coli* K12.

Illustrative examples thereof include ZAP Express {manufactured by Stratagene, *Strategies,* 5, 58 (1992)}, pBluescript II SK(+) {*Nucleic Acids Research,* 17, 9494 (1989)}, Lambda ZAP II (manufactured by Stratagene), λgt10 and λgt11 {DNA Cloning, A Practical Approach, 1, 49 (1985) }, λTriplEx (manufactured by Clontech), λExCell (manufactured by Pharmacia), pT7T318U (manufactured by Pharmacia), pcD2 {*Mol*. *Cell. Biol.,* 3, 280 (1983)}, pUC18 *{Gene,* 33, 103 (1985)}, pAMo {J. Biol. Chem., 268, 22782 - 22787 (1993), alias pAMoPRC3Sc (Japanese published unexamined patent application No. 05-336963) and the like.

As the host microorganism, any microorganism can be used as long as it belongs to the genus *Escherichia coli*. Illustratively, *Escherichia coli* XL1-Blue MRF' {manufactured by Stratagene, *Strategies,* 5, 81 (1992)}, *Escherichia coli* C600 {*Genetics*, 39, 440 (1954)}, *Escherichia coli* Y1088 *{Science,* 222, 778 (1983)}, *Escherichia coli* Y1090 *{Science,* 222, 778 (1983)}, *Escherichia coli* NM522 {*J*. *Mol*. *Biol.,* 166, 1 (1983)}, *Escherichia coli* K802 {*J*. *Mol*. *Biol.,* 16, 118 (1966)}, *Escherichia coli* JM105 {*Gene,* 38, 275 (1985)}, *Escherichia coli* SOLRTM Strain (manufactured by Stratagene), *Escherichia coli* LE392 (Molecular Cloning, Second Edition) and the like can be used.

In addition to the cDNA library prepared by the above method, a commercially available cDNA library can also be used.

For example, any organ cDNA library derived from human, cattle, mouse, rat, rabbit and the like, manufactured by Clontech, Life Technologies Oriental, Inc. and the like, can be utilized as the commercially available cDNA libraries.

### (2) Acquisition of the DNA of the present invention

A cDNA clone having the DNA of the present invention can be selected from the cDNA library prepared in the above step (1), by colony hybridization method or using the isotope- or fluorescence-labeled probe, the plaque hybridization method (Molecular Cloning, Second Edition) or the like.

A fragment obtained by amplifying a part of cDNA by a polymerase chain reaction (to be referred to as PCR hereinafter)-employed method {PCR Protocols, Academic Press (1990)} using primers designed based on a partially determined nucleotide sequence or an oligonucleotide designed based on a partially determined nucleotide sequence can be used as the probe.

When both of the 5'-terminal and 3'-terminal nucleotide sequences of complete cDNA are revealed by EST or the like, primers prepared based on the nucleotide sequences can be used as the primers.

Alternatively, adapter is added to both ends of the cDNA, and PCR is carried out by 5'-RACE (rapid amplification of cDNA ends) and 3'-RACE (rapid amplification of cDNA ends) using primers prepared based on the nucleotide sequence of the adapter and a partially determined nucleotide sequence, whereby obtaining the cDNA fragments containing the region nearer to 5'-terminal than the primer and containing the region nearer to 3'-terminal than the primer {*Proc*. *Natl*. *Acad*. *Sci*. *USA*, 85, 8998 (1988) }.

By connecting the thus obtained cDNA fragments, the full-length DNA of the present invention can be obtained.

Nucleotide sequence of the DNA obtained by the above method can be determined by integrating the DNA fragments, directly or after digestion with appropriate restriction enzymes, into a vector in the usual way and analyzing the product by a generally used nucleotide sequence analyzing method such as the dideoxy method of Sanger *et al*. {*Proc*. *Natl*. *Acad*. *Sci. USA,* 74, 5463 (1997)} or using nucleotide sequence analyzing apparatus such as 373A DNA sequencer (manufactured by Perkin Elmer), or the sequencers manufactured by Pharmacia or LI-COR or the like.

A plasmid p46-1 which contains a DNA fragment comprising the nucleotide sequence represented by SEQ ID NO:2 can be exemplified as a plasmid containing the DNA of the present invention obtained by the above method.

*Escherichia coli* JM109/p46-1 which contains the plasmid p46-1 has been deposited on August 18, 1998, in National Institute of Bioscience and Human Technology, Agency of Industrial Science and Technology (Higashi 1-1-3, Tsukuba, Ibaraki, Japan), as FERM BP-6462.

Also, DNA of interest derived from other tissues or from other animals (e.g., human, rat or the like) can be obtained based on the nucleotide sequence information of the DNA obtained by the above method or by selecting a DNA which hybridizes with the above DNA under stringent conditions.

A plasmid p3-2 which contains a DNA fragment comprised of the nucleotide sequence represented by SEQ ID NO:6 can, for example, be cited as a plasmid containing the DNA of the present invention obtained by such a method.

*Escherichia coli* JM109/p3-2 which contains the plasmid p3-2 has been deposited on August 5, 1999, in National Institute of Bioscience and Human Technology, Agency of Industrial Science and Technology (Higashi 1-1-3, Tsukuba, Ibaraki, Japan), as FERM BP-6830.

The DNA of interest can also be prepared by its chemical synthesis using a DNA synthesizer based on the nucleotide sequence information obtained by the above method. Examples of the DNA synthesizer include a DNA synthesizer manufactured by Shimadzu which employs the thiophosphite method and a DNA synthesizer model 392 manufactured by Perkin Elmer which employs the phosphoamidite method.

The novelty of the thus obtained nucleotide sequence can be confirmed by searching nucleotide sequence databases such as GenBank, EMBL, DDBJ and the like using homology searching programs such as BLAST and the like.

When the thus obtained sequence is novel, already existing genes having the homology can be searched by converting it into amino acid sequence and then searching amino acid sequence databases GenPept, PIR, Swiss-Prot and the like using homology searching programs such as FASTA, FrameSearch and the like.

### (3) Preparation of oligonucleotide of the present invention

Oligonucleotides, such as antisense oligonucleotide, sense oligonucleotide and the like, having partial sequence of the DNA of the present invention can be prepared using the DNA and DNA fragments of the present invention obtained by the method in the usual way or using the aforementioned DNA synthesizer.

Examples of the oligonucleotide include a DNA having a sequence identical to continuous 5 to 60 bases in a nucleotide sequence of the aforementioned DNA or a DNA having a sequence complementary to the above DNA, illustratively, a DNA having a sequence identical to continuous 5 to 60 bases in the nucleotide sequence represented by SEQ ID NO:2 or 6 or a DNA having a sequence complementary to the DNA. When these are used as a sense primer and an antisense primer, the aforementioned oligonucleotides in which the melting temperature (Tm) and the number of bases are not significantly different between both cases are preferable.

As such oligonucleotides, oligonucleotides selected from SEQ ID NO:3, 4, 7 and 8 can, for example, be cited.

In addition, derivatives of these oligonucleotides can also be used as the oligonucleotides of the present invention.

Examples of the oligonucleotide derivatives include an oligonucleotide derivative in which phosphodiester linkage in the oligonucleotide is substituted by phosphorothioate linkage, an oligonucleotide derivative in which phosphodiester linkage in the oligonucleotide is substituted by N3'-P5' phosphoamidate linkage, an oligonucleotide derivative in which ribose and phosphodiester linkage in the oligonucleotide are substituted by peptide-nucleic acid linkage, an oligonucleotide derivative in which uracil in the oligonucleotide is substituted by C-5 propynyluracil, an oligonucleotide derivative in which uracil in the oligonucleotide is substituted by C-5 thiazoleuracil, an oligonucleotide derivative in which cytosine in the oligonucleotide is substituted by C-5 propynylcytosine, an oligonucleotide derivative in which cytosine in the oligonucleotide is substituted by phenoxazine-modified cytosine, an oligonucleotide derivative in which ribose in the oligonucleotide is substituted by 2'-O-propylribose and an oligonucleotide derivative in which ribose in the oligonucleotide is substituted by 2'-methoxyethoxyribose. {*Saibo Kogaku* (Cell Technology), 16, 1463 (1997)}.

### {2} Preparation of polypeptide of the present invention

### (1) Preparation of transformant

In order to produce the polypeptide of the present invention by expressing the DNA of the present invention obtained by the method described in the above section {1} in a host cell, the methods described in Molecular Cloning, Second Edition, Current Protocols in Molecular Biology and the like can be used.

That is, the polypeptide of the present invention can be produced by constructing a recombinant vector into which the DNA of the present invention is inserted downstream of the promoter of an appropriate expression vector, introducing the vector into a host cell, thereby obtaining a transformant capable of expressing the polypeptide of the present invention, and then culturing the transformant.

As the host cell, any bacterial cell, yeast, animal cell, insect cell, plant cell and the like can be used, as long as it can express the gene of interest.

The expression vector to be used is a vector which can be autonomously replicated in the above host cells or can be integrated into chromosome and contains a promoter at a position where the DNA of the present invention can be transcribed.

When prokaryotic organism such as bacteria is used as the host cell, it is preferable that the expression vector for the gene encoding the polypeptide of the present invention can be autonomously replicated in the prokaryotic organism and is a recombinant vector constituted from a promoter, a ribosome binding sequence, the DNA of the present invention and a transcription termination sequence. A gene which regulates the promoter may also be included.

Examples of the expression vector include pBTrp2, pBTac1, pBTac2 (all available from Boehringer-Mannheim), pKK233-2 (Pharmacia), pSE280 (Invitrogen), pGEMEX-1 (Promega), pQE-8 (QIAGEN), pKYP10 (Japanese published unexamined patent application No. 58-110600), pKYP200 {*Agric*. *Biol*. *Chem.,* 48, 669 (1984)}, pLSA1 {*Agric*. *Biol. Chem*., 53, 277 (1989)}, pGEL1 {*Proc*. *Natl*. *Acad*. *Sci*. *USA*, 82, 4306 (1985)}, pBluescript II SK(-) (Stratagene), pTrs32 (FERM BP-5408), pGHA2 (FERM BP-400), pGKA2 (FERM B-6798), pTerm2 (Japanese published unexamined patent application No. 3-22979, US 4686191, US 4939094, US 5160735), pGEX (Pharmacia), pET-3 (Novagen), pSupex, pUB110, pTP5, pC194, pTrxFus (Invitrogen), pMAL-c2 (New England Biolabs) and the like.

The promoter may be any one which can be expressed in host cells such as of *Escherichia coli*, *Bacillus subtilis* and the like. For example, promoters derived from *Escherichia coli,* phage and the like such as *trp* promoter (Ptrp), *lac* promoter (Plac), P_{L} promoter, P_{R} promoter, T7 promoter and the like, and SPO1 promoter, SPO2 promoter, penP promoter and the like can be cited. In addition, artificially designed and modified promoters such as a Ptrp x 2 promoter having two P*trp* promoters in tandem, *tac* promoter, lacT7 promoter, let I promoter and the like can also be used.

As the ribosome binding sequence, it is preferable to use a plasmid in which distance between the Shine-Dalgarno sequence and an initiation codon is adjusted to an appropriate distance (e.g., 6 to 18 bases).

Although a transcription termination sequence is not always necessary for the expression of the DNA of the present invention, it is desirable to arrange the transcription termination sequence just downstream from the structural gene.

Examples of the host cell include microorganisms belonging to the genus *Escherichia,* the genus *Serratia,* the genus *Bacillus*, the genus *Brevibacterium*, the genus *Corynebacterium,* the genus *Microbacterium*, the genus *Pseudomonas* and the like, such as *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coil* No. 49, *Escherichia coli* W3110, *Escherichia coli* NY49, *Serratia ficaria*, *Serratia fonticola*, *Serratia liquefaciens*, *Serratia marcescens, Bacillus subtilis*, *Bacillus amyloliquefaciens, Brevibacterium ammoniagenes*, *Brevibacterium immariophilum* ATCC 14068, *Brevibacterium saccharolyticum* ATCC 14066, *Corynebacterium glutamicum* ATCC 13032, *Corynebacterium glutamicum* ATCC 14067, *Corynebacterium glutamicum* ATCC 13869, *Corynebacterium acetoacidophilum* ATCC 13870, *Microbacterium ammoniaphilum* ATCC 15354, and *Pseudomonas* sp. D-0110.

Regarding the method for introducing the recombinant vector, any method for introducing DNA into the above host cells can be used, and its examples include a method which uses calcium ion {*Proc*. *Natl*. *Acad. Sci*. *USA,* 69, 2110 (1972)}, protoplast method (Japanese published unexamined patent application No. 63-248394), and electroporation method *{Gene,* 17, 107 (1982), *Molecular* & *General Genetics,* 168, 111 (1979)}.

When yeasts are used as the host cell, YEp13 (ATCC 37115), YEp24 (ATCC 37051), YCp50 (ATCC 37419), pHS19, pHS15 and the like can, for example, be used as the expression vector.

Any promoter can be used as long as it can be expressed in yeast and its examples include PHO₅ promoter, PGK promoter, GAP promoter, ADH promoter, gal 1 promoter, gal 10 promoter, heat shock polypeptide promoter, MFα1 promoter, and CUP 1 promoter.

Examples of the host cell include yeasts belonging to the genus *Saccharomyces*, the genus *Schizosaccharomyces,* the genus *Kluyveromyces*, the genus *Trichosporon,* and the genus *Schwanniomyces*, illustratively, *Saccharomyces cerevisiae*, *Schizosaccharomyces pombe*, *Kluyveromyces lactis,* *Trichosporon pullulans*, *Schwanniomyces alluvius, Pichia pastoris* and the like can be cited.

Regarding the method for introducing the recombinant vector, any one of the methods for introducing DNA into yeast, such as electroporation method *{Methods in Enzymology*, 194, 182 (1990)}, spheroplast method {*Proc*. *Natl*. *Acad*. *Sci. USA,* 81, 4889 (1984)}, lithium acetate method {*Journal of Bacteriology,* 153, 163 (1983)} and the like, can be used.

When an animal cell is used as the host, pcDNAI/Amp (manufactured by Invitrogen), pcDNAI, pCDM8 {*Nature*, 329, 840 (1987)}, pAGE107 {Japanese published unexamined patent application No. 3-22979; *Cytotechnology*, 3, 133 (1990)}, pREP4 (manufactured by Invitrogen), pAGE103 {*Journal of Biochemistry*, 101, 1307 (1987)}, pAMo, pAMoA, pAS3-3 (Japanese published unexamined patent application No. 2-227075) and the like can be used as the expression vector.

As a promoter, any promoter capable of driving the expression in animal cells can be used, and its examples include the promoter of IE (immediate early) gene of cytomegalovirus (CMV), the early promoter of SV40 or the promoter of metallothionein, the promoter of retrovirus, the heat shock promoter, the SRα promoter and the like. Furthermore, the enhancer of the IE gene of human CMV may be used together with the promoter.

Examples of the animal cells include mouse myeloma cells, rat myeloma cells, mouse hybridoma cells, Namalwa cells or Namalwa KJM-1 cells as human cells, human fetal kidney cells, human leukemia cells, African green monkey kidney cells, CHO cells as Chinese hamster cells, and HBT5637 (Japanese published unexamined patent application No. 63-299).

Illustratively, SP2/0, NSO and the like can be cited as the mouse myeloma cells, YB2/0 and the like as the rat myeloma cells, HEK293 (ATCC CRL-1573), 293 and the like as the human fetal kidney cells, BALL-1 and the like as the human leukemia cells and COS-1, COS-7 and the like as the African green monkey kidney cells.

Regarding the method for introducing the recombinant vector, any one of the methods for introducing DNA into animal cells can be used, and electroporation method {*Cytotechnology*, 3, 133 (1990)}, calcium phosphate method (Japanese published unexamined patent application No. 2-227075), lipofection method {*Proc*. *Natl*. *Acad*. *Sci*. *USA,* 84, 7413 (1987)}, the method described in *Virology,* 52, 456 (1973) and the like can be cited.

When insect cells are used as the host, the polypeptide can be expressed by the method described, for example, in Baculovirus Expression Vectors, A Laboratory Manual, W.H. Freeman and Company, New York (1992), Molecular Biology, A Laboratory Manual, Current Protocols in Molecular Biology or *Bio/Technology*, 6, 47 (1988).

That is, the polypeptide can be expressed by simultaneously introducing a recombinant gene transfer vector and baculovirus into an insect cell to obtain a recombinant virus in an insect cell culture supernatant and then infecting the insect cell with the recombinant virus.

Examples of the gene transfer vector to be used in this method include pVL1392, pVL1393, and pBlueBacIII (all manufactured by Invitrogen).

Examples of the baculovirus include *Autographa californica* nuclear polyhedrosis virus and the like which can infect insects of the family *Barathra.*

Examples of the insect cells include *Spodoptera frugiperda* oocytes, *Trichoplusia ni* oocytes, and cultured cells derived from silkworm ovaries.

Illustratively, Sf9 and Sf21 (Baculovirus Expression Vectors, A Laboratory Manual) and the like can be cited as the *Spodoptera frugiperda* oocytes, High 5, BTI-TN-5B1-4 (manufactured by Invitrogen) and the like as the *Trichoplusia ni* oocytes and *Bombyx mori* N4 and the like as the cultured cells derived from silkworm ovaries.

Regarding the method for the co-transfer of the aforementioned recombinant gene transfer vector and the aforementioned baculovirus for the preparation of the recombinant virus, calcium phosphate method (Japanese published unexamined patent application No. 2-227075), lipofection method {*Proc*. *Natl*. *Acad*. *Sci*. *USA,* 84, 7413 (1987)} and the like can be exemplified.

Regarding the gene expression method, secretion production, fusion protein expression and the like can be carried out in accordance with the method described in Molecular Cloning, Second Edition, in addition to the direct expression.

When expressed in a yeast cell, an animal cell or an insect cell, glycopolypeptide and glycosylated protein can be obtained.

The polypeptide of the present invention can be produced by culturing a transformant obtained in the aforementioned manner in a medium, thereby effecting formation and accumulation of the polypeptide of the present invention in the resulting culture, and then collecting it from the culture.

In addition, the polypeptide of the present invention can be expressed in the body of a patient by introducing an appropriate expression vector for expressing the polypeptide of the present invention into a cell collected from the body of the patient and then returning the cell into the body.

### (2) Culturing of transformant

The method for culturing the transformant of the present invention in a medium can be carried out in accordance with a usual culturing method for the host.

Regarding the medium for culturing of a transformant obtained using a prokaryote such as *Escherichia coli* or the like or a eucaryote such as yeast or the like as the host, either a natural medium or a synthetic medium may be used as long as it contains materials which can be assimilated by the organism such as carbon sources, nitrogen sources, inorganic salts and the like and can perform culturing of the transformant efficiently.

Examples of the carbon source include those which can be assimilated by the organism, such as glucose, fructose, sucrose, molasses containing them, carbohydrates such as starch, starch hydrolysate and the like, organic acids such as acetic acid, propionic acid and the like and alcohols such as ethanol, propanol and the like.

Examples of the nitrogen source include ammonia, ammonium salts of inorganic acids or organic acids, such as ammonium chloride, ammonium sulfate, ammonium acetate, ammonium phosphate and the like, and other nitrogen-containing compounds, as well as peptone, meat extract, yeast extract, corn steep liquor, casein hydrolysate, soybean meal and soybean meal hydrolysate and various cells obtained by fermentation and digested products thereof.

Examples of the inorganic material include potassium dihydrogen phosphate, dipotassium hydrogen phosphate, magnesium phosphate, magnesium sulfate, sodium chloride, ferrous sulfate, manganese sulfate, copper sulfate, and calcium carbonate.

The culturing is carried out generally under aerobic condition by a shaking culture, submerged agitation aeration culture or the like. The culturing temperature is preferably from 15 to 40°C, and the culturing time is generally from 16 to 96 hours. During the culturing, the medium pH is controlled at 3.0 to 9.0. Adjustment of the medium pH is carried out using an inorganic or organic acid, an alkali solution, urea, calcium carbonate, ammonia and the like.

If necessary, antibiotics such as ampicillin, tetracycline and the like may be added to the medium during the culturing.

When a microorganism transformed with an expression vector using an inducible promoter as the promoter is cultured, an inducer may be added to the medium if necessary. For example, isopropyl-β-D-thiogalactopyranoside or the like may be added to the medium when a microorganism transformed with an expression vector using *lac* promoter is cultured, or indoleacrylic acid or the like may be added to the medium when a microorganism transformed with an expression vector using *trp* promoter is cultured.

Regarding the medium for culturing a transformant obtained using an animal cell as the host, generally used RPMI 1640 medium {*The Journal of the American Medical Association*, 199, 519 (1967)}, Eagle's MEM medium *{Science,* 122, 501 (1952)}, DMEM medium {*Virology*, 8, 396 (1959)}, 199 Medium {*Proceeding of the Society for the Biological Medicine*, 73, 1 (1950)} or any one of these media further supplemented with fetal calf serum can be used.

The culturing is carried out generally at a medium pH of from 6 to 8 and at a temperature of from 30 to 40°C for a period of from 1 to 7 days in the presence of 5% CO₂.

If necessary, antibiotics such as kanamycin, penicillin, streptomycin and the like may be added to the medium during the culturing.

Regarding the medium for culturing a transformant obtained using an insect cell as the host, usually used TNM-FH medium (manufactured by Pharmingen), Sf-900 II SFM medium (manufactured by Life Technologies), ExCell 400 or ExCell 405 (both manufactured by JRH Biosciences), Grace's Insect Medium {Nature, 195, 788 (1962)} or the like can be used.

The culturing is carried out generally at a medium pH of from 6 to 7 and at a temperature of 25 to 30°C for a period of from 1 to 5 days.

In addition, antibiotics such as gentamicin and the like may be added to the medium during the culturing if necessary.

### (3) Isolation and purification of expressed polypeptide

In order to isolate and purify the polypeptide expressed by the aforementioned method from a culture of the aforementioned transformant, usual methods for the isolation and purification of enzymes can be employed.

For example, when the polypeptide of the present invention is expressed in a soluble forms in the cells, after completion of the culturing the cells are recovered by centrifugation, suspended in a buffer of aqueous system and then disrupted using ultrasonic-disrupting machine, French Press, Manton Gaulin homogenizer, dynomill or the like to obtain a cell-free extract.

Purified product can be obtained from a supernatant prepared by centrifuging the cell-free extract, by employing a technique or a combination of techniques for general enzyme isolation and purification methods, such as solvent extraction, salting out with ammonium sulfate or the like, desalting, precipitation with organic solvents, anion exchange chromatography which uses a resin such as diethylaminoethyl (DEAE)-Sepharose, DIAION HPA-75 (manufactured by Mitsubishi Chemical) or the like resin, cation exchange chromatography which uses a resin such as S-Sepharose FF (manufactured by Pharmacia) or the like, hydrophobic chromatography which uses a resin such as butyl-Sepharose, phenyl-Sepharose or the like, gel filtration which uses a molecular sieve, affinity chromatography, chromatofocusing, electrophoresis such as isoelectric focusing or the like.

Also, when the polypeptide is expressed in the cells as an inclusion body, the cells are recovered, disrupted and centrifuged in the same manner, the polypeptide is recovered from the thus obtained precipitated fraction in the usual way and then the inclusion body of the polypeptide is solubilized using a protein denaturing agent. The polypeptide is renatured into normal tertiary structure by diluting with or dialyzing against the solubilized solution which does not contain the protein denaturing agent or contains the agent at a low concentration enough not to denature the polypeptide, and then its purified product is obtained by the aforementioned isolation purification method.

When the polypeptide of the present invention or a derivative thereof such as a glycosylated product is secreted outside the cells, the polypeptide or its derivative such as a sugar chain-added product can be recovered from the culture supernatant.

That is, the purified product can be obtained by treating the culture with centrifugation or the like similar to the above method, thereby obtaining a soluble fraction, and then subjecting the soluble fraction to the aforementioned isolation purification method.

Also, the polypeptide of the present invention can be purified after producing the polypeptide of the present invention as a fusion protein with other protein and then treating the fusion protein with an affinity chromatography which uses a substance having affinity for the fused protein. For example, the polypeptide of the present invention can be purified after producing the polypeptide of the present invention as a fusion protein with protein A and then treating the fusion protein with an affinity chromatography which uses immunoglobulin G, in accordance with the method of Lou *et al*. {*Proc*. *Natl*. *Acad*. *Sci*. *USA,* 86, 8227 (1989), *Genes Dev.,* 4, 1288 (1990)} or the method described in Japanese published unexamined patent application No. 05-336963 or Japanese published unexamined patent application No. 06-823021. Also, the polypeptide of the present invention can be purified after producing the polypeptide of the present invention as a fusion protein with Flag peptide and then treating the fusion protein with an affinity chromatography which uses anti-Flag antibody {*Proc*. *Natl*. *Acad*. *Sci. USA,* 86, 8227 (1989), *Genes Dev*., 4, 1288 (1990)}. In addition, it can also be purified by an affinity chromatography which uses an antibody against the polypeptide itself.

In addition, the polypeptide of the present invention can also be produced by a chemical synthesis method such as the Fmoc method (fluorenylmethyloxycarbonyl method), tBoc method (t-butyloxycarbonyl method) or the like. It can also be synthesized chemically making use of a peptide synthesizer available from companies such as Advanced ChemTech, Perkin-Elmer, Pharmacia, Protein Technology Instrument, Synthecell-Vega, PerSeptive, Shimadzu or the like.

Structural analysis of the thus purified polypeptide of the present invention can be carried out by a method generally used in the protein chemistry, such as the method described in *Idensi kuroningu no tameno tanpakusitu kozokaiseki* (Protein Structure Analysis for Gene Cloning) (edited by H. Hirano, published by Tokyo Kagaku Dojin, 1993).

### {3} Preparation of antibody which recognizes the polypeptide of the present invention

### (1) Preparation of polyclonal antibody

A polyclonal antibody can be prepared using the purified sample of the full-length or a partial fragment of the polypeptide of the present invention obtained by the method of the above section {2} as an antigen and administering it to an animal.

Rabbits, goats, 3- to 20-week-old rats, mice, hamsters and the like can be used as the animal to be administered.

The preferable dosage of the antigen is from 50 to 100 µg per animal.

When a peptide is used, it is preferable to use the peptide as the antigen after binding it to a carrier protein such as keyhole limpet haemocyanin, bovine thyroglobulin or the like by covalent bonding. The peptide to be used as the antigen can be synthesized using a peptide synthesizer.

Administration of the antigen is carried out 3 to 10 times at one- to two-week intervals after the first administration. A blood sample is collected from the venous plexus of the fundus of the eye 3 to 7 days after each administration, and the serum is tested by enzyme immunoassay {*Koso meneki sokuteiho* (Enzyme-linked Immunosorbent Assay) (ELISA) : published by Igaku Shoin, 1976, Antibodies - A Laboratory Manual, Cold Spring Harbor Laboratory Press, 1988} as to whether it is reactive with the antigen used for immunization.

A polyclonal antibody can be obtained by collecting a serum sample from non-human mammal in which the serum showed a sufficient antibody titer against the antigen used for immunization, and isolating and purifying the serum.

Examples of the method for isolating and purifying the antibody include centrifugation, salting out with 40 to 50% saturated ammonium sulfate, caprylic acid precipitation {Antibodies - A Laboratory Manual, Cold Spring Harbor Laboratory, 1988} and a chromatography which uses a DEAE-Sepharose column, an anion exchange column, a protein A- or G-column, a gel filtration column or the like, which may be used alone or in combination.

### (2) Preparation of monoclonal antibody

### (2-1) Preparation of antibody producing cells

A rat whose serum shows a sufficient antibody titer against the antigen used for immunization in the above step (1) is used as the source for antibody producing cells.

The spleen is excised 3 to 7 days after the final administration of the antigen substance to the rat which showed the above antibody titer.

The spleen is cut into pieces in MEM medium (manufactured by Nissui Pharmaceutical). Cells are then loosened using a pair of forceps and centrifuged at 1,200 rpm for 5 minutes, and then the supernatant is discarded.

Splenocytes in the thus obtained precipitation fraction are treated with Tris-ammonium chloride buffer (pH 7.65) for 1 to 2 minutes for removing erythrocytes and then washed three times with MEM medium, and the thus obtained splenocytes are used as the antibody-producing cells

### (2-2) Preparation of myeloma cells

Established cell lines obtained from mouse or rat are used as the myeloma cells. For example, 8-azaguanine-resistant mouse (BALB/c-derived) myeloma cell strains such as P3-X63Ag8-U1 (P3-U1) {*Curr*. *Topics Microbiol*. *Immunol*., 81, 1 (1978), *Eur*. *J. Immunol*., 6, 511 (1976)}, SP2/O-Ag14 (SP-2) *{Nature,* 276, 269 (1978) }, P3-X63-Ag8653 (653) {*J. Immunol*., 123, 1548 (1979) }, P3-X63-Ag8 (X63) *{Nature,* 256, 495 (1975)} and the like can be used. These cell lines are subcultured in an 8-azaguanine medium {prepared by supplementing RPMI-1640 medium with glutamine (1.5 mM), 2-mercaptoethanol (5 x 10⁻⁵ M), gentamicin (10 µg/ml) and fetal calf serum (FCS) (manufactured by CSL, 10%) and further supplementing the resulting medium (referred to as "normal medium" hereinafter) with 8-azaguanine (15 µg/ml)}, wherein the culturing is carried out in the normal medium 3 to 4 days before cell fusion to thereby ensure a cell number of 2 x 10⁷ cells or more on the day of cell fusion.

### (2-3) Preparation of hybridoma

The antibody-producing cells obtained in the step (2-1) and the myeloma cells obtained in the step (2-2) are respectively washed thoroughly with MEM medium or PBS (1.83 g of disodium hydrogenphosphate, 0.21 g of potassium dihydrogenphosphate and 7.65 g of sodium chloride, 1 liter of distilled water, pH 7.2) and mixed in a proportion of antibody producing cells : myeloma cells = 5:1 to 10:1, and the mixture is centrifuged at 1,200 rpm for 5 minutes and then the supernatant is discarded.

Cells in the thus obtained precipitation fraction are thoroughly loosened, a mixture of 2 g of polyethylene glycol-1000 (PEG-1000), 2 ml of MEM and 0.7 ml of dimethyl sulfoxide (DMSO) is added to the cells in an amount of from 0.2 to 1 ml per 10⁸ antibody producing cells while stirring at 37°C, and then 1 to 2 ml of MEM medium is added several times at 1 to 2 minute intervals.

After the addition, the whole volume is adjusted to 50 ml by adding MEM medium. After centrifugation of the thus prepared solution at 900 rpm for 5 minutes, the supernatant is discarded.

Cells in the thus obtained precipitation fraction are gently loosened and then suspended by pipetting in 100 ml of HAT medium {prepared by supplementing the normal medium with hypoxanthine (10⁻⁴ M), thymidine (1.5 x 10⁻⁵ M) and aminopterin (4 x 10⁻⁷ M) }.

The suspension is dispensed in 100 µl portions into each well of a 96-well culture plate and cultured at 37°C for 7 to 14 days in a 5% CO₂ incubator.

After the culturing, a portion of the culture supernatant is sampled from each well and subjected to enzyme immunoassay described, for example, in {Antibodies - A Laboratory Manual, Cold Spring Harbor Laboratory Press, Chapter 14 (1988)}, and hybridoma strains which specifically react with the polypeptide of the present invention are selected.

The following method can be cited as an illustrative example of the enzyme immunoassay.

The purified sample of the full-length or partial fragment of the peptide of the present invention used as the antigen in carrying out the immunization is coated on an appropriate plate, allowed to react with a first antibody, namely a hybridoma culture supernatant or the purified antibody obtained in the following step (2-4), further allowed to react with a second antibody, namely an anti-rat immunoglobulin antibody labeled with biotin, an enzyme, a chemiluminescence substance, a radioactive compound or the like and then subjected to the reaction corresponding to the used marker, and those which react specifically with the polypeptide of the present invention are selected as hybridoma strains that produce monoclonal antibody against the polypeptide of the present invention.

Using the hybridoma strains, cloning is repeated twice by the limiting dilution method {using HT medium (a medium prepared by removing aminopterin from HAT medium) for the first cloning and the normal medium for the second}, and those for which a high antibody titer is constantly observed are selected as hybridoma strains that produce monoclonal antibody against the polypeptide of the present invention.

### (2-4) Preparation of monoclonal antibody

The hybridoma cells capable of producing monoclonal antibody against the polypeptide of the present invention obtained in the step (2-3) are injected into the abdominal cavity of 8 to 10-week-old mice or nude mice treated with pristane {by intraperitoneal administration of 0.5 ml of 2,6,10,14-tetramethylpentadecane (pristane) followed by 2 weeks of feeding} at a dose of 5 x 10⁶ to 20 x 10⁶ cells per animal. The hybridoma causes ascites tumor in 10 to 21 days.

The ascitic fluid is collected from the ascites tumor-bearing mice and centrifuged at 3,000 rpm for 5 minutes to remove solid matter.

The monoclonal antibody can be obtained by purifying it from the thus obtained supernatant by the same method used in the purification of polyclonal antibody.

Determination of the subclass of the antibody is carried out using a mouse monoclonal antibody typing kit or a rat monoclonal antibody typing kit. The amount of protein is calculated by the Lowry method or from the absorbance at 280 nm.

### {4} Measurement of nucleoside transporter activity of the polypeptide of the present invention

The polypeptide of the present invention expressed using *Escherichia coli*, an yeast, an insect cell, an animal cell or the like as the host by the method described in {2} or expressed in Xenopus oocyte by a micro-injection method using a DNA or a cRNA prepared *in vitro* is used in the following nucleoside transporter activity measurement {*Methods in Enzymology,* 207, 225 (1992), *Methods in Enzymology,* 254, 458 (1995)}.

Alternatively, a preparation in which the polypeptide of the present invention expressed by the above method is reconstructed on a liposome membrane (lipid bilayer) by a known method can also be used in the following measurement {*J*. *Biol*. *Chem*., 272, 617 (1997), *Biochim*. *Biophys*. *Acta*., 1024, 289 (1990), *J. Biol*. *Chem*., 252, 7384 (1997)}.

The nucleoside transporter activity can be obtained by measuring incorporation of a fluorescence-labeled or isotope-labeled nucleoside, such as {5,6-³H}uridine, {¹⁴C}uridine, {¹⁴C}adenosine or the like, into cell or liposome in the presence of the polypeptide of the present invention {*Biochim*. *Biophys*. *Acta*., 649, 769 (1981), *Nature Medicine,* 3, 89 (1997), *Biochem*. *J*., 315*,* 329 (1996), *Biochim*. *Biophys*. *Acta.,* 1024, 289 (1990)}.

### {5} Screening and identification and use as a treating agent of agonist or antagonist of the polypeptide of the present invention

Using a cell or liposome which can be used in the activity measurement of the above section {4} or a tissue or cell in which expression of the polypeptide was confirmed by the method of {7} which will be described later, each test sample is added and the transporter activity is measured by the method of {4}.

By comparing the transporter activity of the polypeptide of the present invention {*Biochim*. *Biophys. Acta*., 649*,* 769 (1981), *Nature Medicine*, 3, 89 (1997), *Biochem*. *J*., 315, 329 (1996), *Biochim*. *Biophys*. *Acta.,* 1024, 289 (1990)} in the presence and absence of a test sample, a substance which increases the transporter activity (agonist) and a substance which inhibits the same (antagonist) can be screened from the samples.

Alternatively, agonist and antagonist of the polypeptide of the present invention can be screened also using the following method.

Using the aforementioned cell or tissue expressing the polypeptide of the present invention, cell membrane prepared therefrom, purified polypeptide of the present invention or a partial fragment of the polypeptide, the presence or absence of binding of a labeled compound thereto is checked {*Biochem*. *J*., 327, 31 (1997), *Life Science,* 59, 2051 (1996), *Biochem*. *J.,* 300, 407 (1994)}.

Examples of the labeled compound include isotope-labeled nucleoside analogues such as {³H}NBMPR and the like and nucleoside transporter inhibitors having an isotope-labeled non-nucleoside structure {*Current Medicinal Chemistry,* 4, 35 (1997)}.

Using the labeled compound whose binding is confirmed, each test sample is added under the same conditions described in the above, and binding amount of the labeled compound is measured in the same manner as described above.

By comparing the binding amount of the labeled compound in the presence and absence of a test sample, agonist and antagonist can be screened from the samples.

Examples of the test samples include synthetic compounds, naturally occurring proteins, artificially synthesized proteins, peptides, saccharides, lipids and modified products and derivatives thereof, as well as urine, body fluids, tissue extracts, cell culture supernatants and cell extracts of mammals (e.g., mouse, rat, guinea pig, hamster, swine, sheep, bovine, horse, dog, cat, monkey, human and the like), and also non-peptide compounds, fermentation products, extracts of plants and other organisms and the like.

Although it is possible to use the agonist or antagonist of the polypeptide of the present invention obtained by the above method directly as a therapeutic agent, it is preferable in general to use it as a pharmaceutical preparation produced by an optional method well known in the technical field of pharmaceutics, by mixing it with one or more pharmacologically acceptable carriers.

Regarding the administration method of the therapeutic agent, it is preferable to use the most effective method in carrying out the treatment, and methods by oral administration or by parenteral administration such as buccal, airway, rectal, subcutaneous, intramuscular, intravenous or the like can be used.

Examples of the dosage form of the therapeutic agents include ointments, sprays, capsules, tablets, granules, syrups, emulsions, suppositories, injections, and tapes.

Examples of the preparations suited for oral administration include emulsions, syrups, capsules, tablets, powders, granules and the like.

Liquid preparations such as emulsions and syrups can be produced using water, saccharides such as sucrose, sorbitol, fructose and the like, glycols such as polyethylene glycol, propylene glycol and the like, oils such as sesame oil, olive oil, soybean oil and the like, antiseptics such as p-hydroxybenzoic acid esters and the like and flavors such as strawberry flavor, peppermint and the like, as the additive agents.

Capsules, tablets, powders, granules and the like can be produced using excipients such as lactose, glucose, sucrose, mannitol and the like, disintegrators such as starch, sodium alginate and the, lubricants such as magnesium stearate, talc and the like, binders such as polyvinyl alcohol, hydroxypropylcellulose, gelatin and the like, surfactants such as fatty acid ester and the like, and plasticizers such as glycerol and the like, as the additive agents.

Examples of the preparations suitable for parenteral administration include injections, suppositories, sprays and the like.

Injections can be prepared for example using a carrier comprised of a salt solution, a glucose solution or a mixture thereof.

Suppositories can be produced for example using a carrier such as cacao butter, hydrogenated fat, carboxylic acid or the like.

Regarding sprays, the agonist or antagonist obtained in the above can be used directly as sprays. However, preferable sprays are prepared using a carrier which does not stimulate buccal and airway mucous membranes of the recipient subject and can facilitate absorption of the compound by dispersing it as fine particles.

Examples of the carrier include lactose, glycerol and the like.

Depending on the properties of the agonist or antagonist obtained in the above and of the carrier, pharmaceutical preparations such as aerosols, dry powders and the like can be prepared.

The components exemplified as additive agents of oral preparations can also be added to these parenteral preparations.

The dose or frequency of administration vary depending on the intended therapeutic effect, administration method, treating period, age, body weight and the like but is generally from 10 µg/kg to 8 mg/kg per day for an adult.

### {6} Screening and identification of compound which regulates expression of the polypeptide of the present invention (to be referred to as "expression-regulating compound" hereinafter)

### (1) Screening and identification of expression-regulating compound using the antibody of the present invention

After contacting cells expressing the polypeptide of the present invention with a test sample, an expression-regulating compound existing in the cells or a cell culture supernatant can be screened and identified using the antibody of the present invention.

Any of cells, cell strains and tissues expressing the polypeptide of the present invention can be used as the cell.

Cells, cell strains or tissues can also be used in which expression of the polypeptide has been observed using the antibody described in the following section {7} for immunologically detecting.

As a suitable cell strain, a human kidney-derived HEK293 cell (ATCC CRL-1573) can be cited, for example.

Examples of the test sample include those which were exemplified in the above section {5}.

Cells expressing the polypeptide of the present invention are suspended in a medium in which the cells can grow, each test sample is added to the medium so as to contact it with the cells, and then amount of the polypeptide expressed by the cells is determined using the antibody of the present invention. Regarding the determination method, the immunocytochemical staining method described in the following can be exemplified.

Cultured adherent cells are washed with PBS buffer, 3 ml of PBS buffer containing 0.05% of trypsin and 0.02% of EDTA (ethylenediaminetetraacetic acid) is added, and then after removing excess solution, incubated at 37°C for 5 minutes to detach the cells from the flask.

In the case of the suspension cells, cultured cells can be used as such.

Cells to be used in the immunocytochemical staining are suspended in, for example, a buffer for immunocytochemcial staining (PBS containing 1% BSA, 0.02% EDTA and 0.05% sodium azide) and dispensed into a round bottom 96-well plate in a portion of from 1 x 10⁵ to 20 x 10⁵ cells per well.

A monoclonal antibody of the present invention is dispensed into the plate.

Examples of the monoclonal antibody include culture supernatant of the culture of hybridoma obtained in {3}-(2-3) capable of producing the monoclonal antibody of the present invention and the purified monoclonal antibody obtained in {3}-(2-4). An antibody prepared by labeling the monoclonal antibody can also be used.

Examples of the antibody prepared by labeling the monoclonal antibody include a biotin-labeled antibody.

The biotin-labeled antibody can be prepared by a known method (Enzyme Immunoassay: published by Gakusai Kikaku, 1985).

The above antibody is diluted to a concentration of from 0.1 to 50 µg/ml using the buffer for immunocytochemical staining or the buffer for immunocytochemical staining containing 10% of an animal serum.

The diluted antibody is dispensed in a portion of from 20 to 500 µl/well and allowed to stand with ice-cooling for 30 minutes.

When unlabeled antibody is used, the cells are washed by adding the buffer for immunocytochemical staining to the aforementioned plate, the buffer for immunocytochemical staining containing approximately from 0.1 to 50 µg/ml of an anti-mouse immunoglobulin antibody or anti-rat immunoglobulin antibody labeled with a fluorescence dye such as FITC (fluorescein isothiocyanate), phycoerythrin or the like is dispensed in a portion of approximately from 50 to 500 µl/well, and then the cells are allowed to stand with ice-cooling for 30 minutes in the dark.

When the monoclonal antibody labeled with biotin is used, streptoavidin labeled with a fluorescent dye such as FITC, phycoerythrin or the like is dispensed in a portion of approximately from 50 to 500 µl/well into the aforementioned plate and then allowed to stand with ice-cooling for 30 minutes in the dark.

In both cases, after placement, the cells are thoroughly washed by adding the buffer for immunocytochemical staining to the plate and then analyzed by a fluorescence microscopy, a cell sorter or the like.

By searching for a test sample capable of increasing or decreasing amount of the polypeptide of the present invention in comparison with the system in which the test sample is not added, an expression-regulating compound can be identified.

### (2) Screening and identification using transcription product determination system of the polypeptide gene of the present invention

After contacting cells which express the polypeptide of the present invention or mRNA encoding the polypeptide with each test sample, an expression-regulating compound can be screened and identified by determining amount of the mRNA.

Examples of the cells expressing the polypeptide of the present invention or mRNA of the polypeptide and the test samples include those described in {5} and {6}-(1).

Cells which express the polypeptide of the present invention or mRNA encoding the polypeptide are suspended in a medium in which the cells can grow, each test sample is added to the medium so as to contact the cells, and then amount of the mRNA expressed by the cells is determined using the conventional Northern hybridization method, the RNA-dot blot hybridization method, the RT-PCR method or the like.

Examples of the probes which can be used for the hybridization method and the like and the primers which can be used for the RT-PCR method and the like include gene fragments which encode the polypeptide of the present invention.

Illustratively, an oligonucleotide having a sequence identical to continuous 5 to 60 bases in the nucleotide sequence described in SEQ ID NO:2 or 6 or an oligonucleotide having a sequence complementary to the former oligonucleotide can be used suitably.

By searching for a test sample capable of increasing or decreasing amount of the mRNA encoding the polypeptide of the present invention in comparison with the system in which the test sample is not added, an expression-regulating compound can be identified.

### (3) Screening and identification using reporter gene

A transformant transformed with a plasmid containing a DNA in which a reporter gene is connected to the downstream of a region which regulates transcription of a gene that encodes the polypeptide of the present invention (to be referred to as "transcription regulation region" hereinafter) is contacted with each test sample, and then an expression-regulating compound can be screened and identified by determining amount of the expressed polypeptide encoded by the reporter gene.

In general, the transcription regulation region is contained in the 5'-upstream region of genes in most cases. The 5'-upstream region of the gene encoding the polypeptide of the present invention can be prepared by, for example, using Genome Walker kits (manufactured by Clontech). Also, a fragment obtained by digesting this region into an appropriate length using appropriate restriction enzymes can be used as the transcription regulation region.

Any gene can be used as the reporter gene as long as the translation product of the gene is stable in cells and existing amount of the translation product can be easily determined, and its examples include chloramphenicol acetyltransferase (CAT), β-galactosidase (β-gal), luciferase (luc), and green fluorescent protein (GFP).

Examples of the host cell into which a reporter plasmid containing the transcription regulation region is to be introduced include any type of cell, but preferably, a cell strain in which expression of the polypeptide of the present invention or mRNA encoding the polypeptide is confirmed as described in {6}-(1).

Examples of the test samples include those described in the above section {5}.

Using a plasmid prepared by connecting the reporter gene to the downstream of the transcription regulation region by a usual method, the host cell is transformed by a conventional method.

Alternatively, a cell strain in which a part of the gene encoding the polypeptide of the present invention is substituted by a reporter gene can be obtained by preparing a gene targeting vector in which a positive selection marker (G418 resistance gene or the like) and a negative selection marker (thymidine kinase gene of herpes simplex virus, diphtheria toxin A fragment gene or the like) are connected *{Nature,* 336, 348 (1988), *Analytical Biochemistry*, 214, 77 (1993), Gene Targeting, The Practical Approach Series, IRL Press (1993)}.

The transformants are suspended, for example, in a medium in which the cells can grow, each test sample is added to the medium so as to contact it with the cells, and then amount of the polypeptide encoded by the reporter gene expressed in the cells is detected and determined using an appropriate method for the polypeptide.

Examples of the detection and determination method include the method described for example in Molecular Cloning, Second Edition, Chapter 16, p. 60 when the reporter gene is CAT, the method described for example in Molecular Cloning, Second Edition, Chapter 16, p. 66 when the reporter gene is β-gal, the method described for example in *Jikken Igaku* (Experimental Medicine), Supplement, Biomanual Series 4, Gene Introduction and Expression Analysis Method, 81 (1994) when the reporter gene is luc and the method described for example in *Proc. Natl*. *Acad*. *Sci. USA,* 94, 4653 (1997) when the reporter gene is GFP.

By searching for a test sample capable of increasing or decreasing amount of the polypeptide encoded by the reporter gene in comparison with the system in which the test sample is not added, an expression-regulating compound can be identified.

### {7} Use of the DNA, polypeptide, antibody, agonist, antagonist and expression-regulating compound of the present invention

(1) The DNA of the present invention can be used for the detection or determination of mRNA of the polypeptide gene of the present invention in tissues of mammals and cells derived from mammals, by carrying out Northern hybridization to RNA extracted from the tissues and cells in the same manner as described in {1}-(1) using the DNA as the probe. By comparing expressed amounts of mRNA in various types of tissues, distribution of expression of the polypeptide of the present invention in tissues can be known.

According to the present invention, the term mammals means animals such as human, mouse, rat, guinea pig, hamster, swine, sheep, bovine, horse, dog, cat, monkey and the like.

(2) The oligonucleotide of the present invention can be used for the detection and determination of mRNA encoding the polypeptide of the present invention, by carrying out RT-PCR {reverse transcription PCR; PCR Protocols (1990)} of RNA extracted from tissues of mammals and cells derived from mammals in the same manner as described in {1}-(1) using it as a specific primer of the DNA of the present invention.

The mRNA determination method can be used, for example, in the diagnosis of pathological states in which the gene is concerned and the prediction of the effect of cytotoxic nucleoside derivatives (e.g., antitumor agents and antiviral agents).

By determining the mRNA in model animals of various pathological states, importance of the gene product in pathological states can be revealed. Also, drugs can be evaluated by comparing expressed amounts of the mRNA in the presence and absence of each drug.

(3) By carrying out *in situ* hybridization {*Methods in Enzymology,* 254, 419 (1995)} of tissue sections of mammals using the oligonucleotide of the present invention as the probe, more detailed distribution of expression of the polypeptide of the present invention in tissues, such as identification of the cells which express the polypeptide of the present invention, can be known.

Information as to which tissues and cells are expressing the polypeptide of the present invention and information concerning what type of stimulation changes expression amount in cells are useful in analyzing participation of the polypeptide of the present invention in physiological functions and pathological states.

(4) By carrying out Southern hybridization {Molecular Cloning, Second Edition} to genomic DNA using the DNA of the present invention as the probe, mutation of the gene encoding the polypeptide of the present invention can be detected.

The detection of mutation renders possible diagnosis of diseases having a possibility that mutation in this gene is a cause of the disease, such as hypertension, ischemic heart disease, glomerular nephritis, pancreatitis and the like.

(5) By inhibiting transcription of the gene encoding the polypeptide of the present invention or translation of the mRNA {Kagaku (Chemistry), 46, 681 (1991), *Bio/Technology*, 9, 358 (1992)} using the antisense oligonucleotide (RNA/DNA) of the present invention, it can be used in the prevention and treatment of diseases having a possibility that the gene is concerned in the onset of diseases, such as hypertension, ischemic heart disease, nephritis, pancreatitis and the like.

Application is also expected for immune response accompanied by organ transplantation, an analgesic, an anti-platelet agent, for reduction of side effects at the time of chemotherapy, an agent for increasing activity of an antiviral agent or a malignant tumor treating agent, treatment or prevention of cerebral disorder at the time of stroke and the like.

The aforementioned antisense oligonucleotide of the present invention is designed and prepared based on an oligonucleotide having a sequence complementary to continuous 5 to 60 bases in a nucleotide sequence of DNA encoding the polypeptide of the present invention, preferably a nucleotide sequence complementary to 5 to 60 bases existing in the translation initiation region of DNA encoding the polypeptide of the present invention, and administered to the living body.

The medicament containing the DNA of the present invention can be prepared using a method similar to the preparation method for the pharmaceutical preparations of the agonist or antagonist of the polypeptide of the present invention described in the aforementioned section {5}, and the thus prepared pharmaceutical preparations can be administered by the same method as the case of the section {5}.

(6) The polypeptide of the present invention can be obtained by the method described in the section {2} using the DNA of the present invention.

Regarding the use of the polypeptide of the present invention, a preventive agent or a therapeutic agent for ischemic heart disease, cerebral disorder at the time of stroke, immune response accompanied by organ transplantation, malignant tumor, nephritis, pancreatitis or hypertension can be considered. Its applications as an analgesic, an antiplatelet agent, an agent for increasing activity of an antiviral agent or a malignant tumor treating agent and an agent for reducing side effects at the time of chemotherapy can also be expected.

The medicament containing the polypeptide of the present invention can be prepared using a method similar to the preparation method of pharmaceutical preparations of the agonist or antagonist of the polypeptide of the present invention described in the aforementioned section {5}, and the thus prepared pharmaceutical preparations can be administered by the same method as the case of the section {5}.

(7) The oligonucleotide of the present invention can be used in gene therapy as a vector for gene therapy, by inserting it as a single-stranded or double-stranded chain into a viral vector such as retrovirus, adenovirus, adeno-associated virus or the like, or other vector.

(8) An antibody against the polypeptide of the present invention can be produced by the method described in {3} using the polypeptide of the present invention as the antigen.

Using the antibody against the polypeptide of the present invention, the polypeptide of the present invention can be detected or determined immunologically.

Illustratively, detection methods such as the ELISA method which uses microtiter plates, the immunocytochemcial staining by enzyme-labeled antibody method or the fluorescent antibody method, the Western blotting method and the like can be used.

Illustratively, a sandwich ELISA method which uses two types monoclonal antibodies recognizing different epitopes, among antibodies which react with the polypeptide of the present invention in a liquid phase, and a radioimmunoassay which uses an antibody capable of recognizing the polypeptide of the present invention and the polypeptide of the present invention labeled with radioisotope such as ¹²⁵I or the like can be employed.

The antibody of the present invention can also be applied to immunohistological staining which uses pathologic tissue sections.

By immunologically detecting or determining the polypeptide of the present invention existing in cells or tissues of a normal person or patient using the antibody of the present invention, comparing its amounts between the normal person and patient and then examining whether the expression amount is changed, the antibody of the present invention can be used for the diagnosis of pathological states of diseases such as ischemic heart disease, hypertension and the like as well as the prediction of the effect of cytotoxic nucleoside derivatives (e.g., antitumor agents and antiviral agents).

Also, by immunologically detecting or determining the polypeptide existing in tissues and cells of model animals of various pathological states using the antibody of the present invention and comparing the result with normal animals, the importance of the polypeptide in pathological states can be revealed. In addition, agents can be evaluated by comparing expressed amounts of the polypeptide in the presence and absence of each agent.

(9) By administering an antibody which inhibits a function (transporter activity) of the polypeptide of the present invention, treatment or prevention of ischemic heart disease, cerebral disorder at the time of stroke, immune response accompanied by organ transplantation, malignant tumor, nephritis, pancreatitis or hypertension, as well as increased activity of an antiviral agent or a malignant tumor treating agent, analgesic activity, inhibition of platelet aggregation, reduction of side effects at the time of chemotherapy and the like, are also be expected.

The medicament containing the antibody of the present invention can be prepared using a method similar to the preparation method of pharmaceutical preparations of the agonist or antagonist of the polypeptide of the present invention described in the aforementioned section {5}, and the thus prepared pharmaceutical preparations can be administered by the same method as the case of the section {5}.

(10) The agonist and antagonist of the present invention and the compound which regulates expression of the polypeptide gene of the present invention are expected to be effective for the treatment or prevention of ischemic heart disease, cerebral disorder at the time of stroke, immune response accompanied by organ transplantation, malignant tumor, nephritis, pancreatitis or hypertension, as well as for the increased activity of an antiviral agent or a malignant tumor treating agent, analgesic activity, inhibition of platelet aggregation, reduction of side effects at the time of chemotherapy and the like.

### Brief Description of the Drawings

Fig. 1 is a figure showing construction steps and restriction enzyme map of plasmid p46-1.

Fig. 2 is a figure showing a comparison of amino acid sequence of the novel human transporter (hENTR1) encoded by p46-1 with amino acid sequences of human es type transporter hENT1 with human ei type transporter hENT2.

The portions shown by asterisk indicate amino acid residues which are identical to each other. (Amino acid residues are shown by the single letter code.)

Fig. 3 is a figure showing a comparison of regions considered to be transmembrane regions (underlined portions) in the amino acid sequence of hENT1 and amino acid sequences of hENT2 and hENTR1. The portions shown by asterisk indicate amino acid residues which are identical to each other. (Amino acid residues are shown by the single letter code.)

Fig. 4 is a figure showing a comparison of hydrophobic plots based on the amino acid sequences of the novel human transporter (hENTR1) encoded by p46-1, human es type transporter hENT1 and human ei type transporter hENT2.

Fig. 5 is a figure showing a result of Northern hybridization carried out on a poly(A)⁺ RNA filter {filter of Human Multiple Tissue Northern Blots (manufactured by Clontech)} of the human heart, brain, placenta, lung, liver, skeletal muscle, kidney and pancreas using a partial sequence (about 1 kb) of the novel human transporter (hENTR1) cDNA as the probe.

Fig. 6 is a figure showing construction steps and restriction enzyme map of plasmid p3-2.

Fig. 7 is a figure showing a comparison of homology of the amino acid sequence of novel human transporter (hENTR1) with the amino acid sequence of novel rat transporter (rENTR1). The asterisk indicates amino acid residues which are identical to each other and the period indicates amino acid residues which are homologous to each other. (Amino acid residues are shown by the single letter code.)

Fig. 8 is a figure showing construction steps and restriction enzyme map of plasmid pRENTR1-Nor.

Fig. 9 is a figure showing a result of Northern hybridization carried out on a poly(A)⁺ RNA filter {filter of rat Multiple Tissue Northern Blots (manufactured by Clontech)} of the rat heart, brain, spleen, lung, liver, skeletal muscle, kidney and testis using a partial sequence (about 0.4 kb) of the novel rat transporter (rENTR1) cDNA as the probe.

Fig. 10 is a figure showing a result of Northern hybridization carried out on a poly(A)⁺ RNA filter {filter of mouse Multiple Tissue Northern Blots (manufactured by Clontech)} of the mouse heart, brain, spleen, lung, liver, skeletal muscle, kidney and testis using a partial sequence (about 0.4 kb) of the novel rat transporter (rENTR1) cDNA as the probe.

### {Description of the Reference Numerals and Signs}

kb: kilobase pairs
Ap: ampicillin resistance gene
knt: kilonucleotides

### Best Mode for Carrying Out the Invention

In the following, the present invention is described with illustrative Examples, but the scope of the invention is not restricted thereby.

### Example 1 Cloning of hENT1-related protein (hENTR1) cDNA

### (1) Cloning from human fetal kidney-derived cDNA library

Unless otherwise noted, known techniques described in Molecular Cloning Second Edition were used as the gene manipulation techniques.

The 5'-terminal side DNA primer shown by SEQ ID NO:3 was designed and synthesized based on an EST sequence (Genbank, ACCESSION R07250) having homology with hENT1 {*Nature Medicine,* 3, 89 (1997)}. In the same manner, the 3'-terminal side DNA primer shown by SEQ ID NO:4 was designed and synthesized based on an EST sequence (Genbank, ACCESSION AA608799).

A 200 pmol portion of the primer shown by SEQ ID NO:3 or SEQ ID NO:4 was dissolved in 20 µl of a buffer containing 50 mM Tris-HCl (pH 8.0), 10 mM magnesium chloride, 5 mM dithiothreitol and 5 mM adenosine triphosphate, and 10 units of T4 polynucleotide kinase (manufactured by Takara Shuzo) were added, and the mixture was subjected to 2 hours of phosphorylation reaction at 37°C, thereby two types of phosphorylated primers were obtained.

PCR was carried out under the following conditions using 50 µl of a reaction solution containing 0.2 µM of each of the thus obtained two types of phosphorylated primers, 2 µl of a human fetal kidney-derived cDNA library (manufactured by Clontech, trade name: Human Fetal Kidney 5'-STRETCH PLUS cDNA Library), a dNTP (dATP, dGTP, dCTP, dTTP) mixture having 200 µM of each component, 2.5 units of TaKaRa Ex Taq (manufactured by Takara Shuzo) and 1 x Ex Taq buffer (Mg plus) .

That is, using TaKaRa PCR Thermal Cycler 480, the reaction solution was heated at 95°C for 2 minutes, subjected to 35 cycles of the reaction using steps of 1 minute at 94°C, 1 minute at 60°C and 1 minute at 72°C as one cycle and then heated at 72°C for 8 minutes.

A 5 µl portion of the thus obtained PCR reaction solution was applied to an agarose electrophoresis to confirm that the predicted DNA fragment of about 1 kb was amplified.

After the confirmation, the amplified DNA fragment was eluted with sterilized water from the PCR reaction solution using Wizard DNA Clean-UP System (manufactured by Promega), thereby the purified DNA fragment was obtained.

The DNA fragment was dissolved in 100 µl of a buffer containing 33 mM of Tris-acetate (pH 7.9), 66 mM potassium acetate, 10 mM magnesium acetate, 0.5 mM dithiothreitol, a dNTP mixture containing 100 µM each component and 0.01% bovine serum albumin, and 5 units of T4 DNA polymerase (manufactured by Takara Shuzo) were added, and the mixture was subjected to blunting by incubating at 37°C for 15 minutes.

The blunt-ended DNA fragment was recovered from the reaction solution using Wizard DNA Clean-UP System (manufactured by Promega).

In order to insert the DNA fragment into a vector, the vector was prepared under the following conditions.

A 5 µg portion of pBluescript II SK(-) (manufactured by STRATAGENE) was digested with 15 units of *Eco*RV (manufactured by Takara Shuzo) at 37°C for 2 hours in 30 µl of a buffer containing 10 mM Tris-HCl (pH 7.5), 10 mM magnesium chloride, 100 mM sodium chloride and 1 mM dithiothreitol.

Digested DNA fragments of pBluescript II SK(-) were recovered from the reaction solution by ethanol precipitation.

The dephosphorylation reaction of the DNA fragment was carried out at 60°C for 30 minutes using 0.5 unit of alkaline phosphatase (manufactured by Takara Shuzo; derived from *E*. *coli* C75) in 30 µl of a buffer containing 50 mM Tris-HCl (pH 9.0) and 1 mM magnesium chloride.

The thus obtained reaction solution was subjected to an agarose electrophoresis to recover a DNA fragment of about 3 kb.

The DNA fragment was purified using a QIAEX II gel extraction kit (manufactured by QIAGEN) in accordance with the manual attached.

A 50 ng portion of the blunt-ended DNA fragment recovered in the above and 50 ng of the EcoRV- and alkaline phosphatase-treated pBluescript II SK(-) fragments were dissolved in 20 µl of a buffer containing 66 mM Tris-HCl (pH 7.5), 6.6 mM magnesium chloride, 10 mM dithiothreitol and 0.1 mM adenosine triphosphate followed by adding 175 units of T4 DNA ligase (manufactured by Takara Shuzo), and the mixture was subjected to 16 hours of ligation reaction at 16°C.

Using the recombinant plasmid DNA obtained by the reaction, an *E. coli* DH5α was transformed in the usual way to obtain a plasmid pHENTlhomologue.

As a result of restriction enzyme analysis, it was revealed that one *Xba*I site is present in the DNA fragment contained in the plasmid pHENTlhomologue and a fragment of about 390 bp is cut out from the *Xba*I site in the insert DNA fragment and the *Xba*I site derived from the multicloning site of the vector pBluescript II SK(-).

A 30 µg portion of pHENTlhomologue was dissolved in 50 µl of a buffer containing 10 mM Tris-HCl (pH 7.5), 10 mM magnesium chloride, 50 mM sodium chloride, 0.01% bovine serum albumin and 1 mM dithiothreitol, and 40 units of *Xba*I (manufactured by Takara Shuzo) were added to carry out 4 hours of the digestion reaction at 37°C.

The reaction solution was subjected to an agarose electrophoresis to recover a DNA fragment of about 390 bp.

The DNA fragment was purified using a QIAEX II gel extraction kit (manufactured by QIAGEN) in accordance with the manual attached.

The thus purified DNA fragment was labeled with horseradish peroxidase using the ECL direct nucleic acid labeling detecting system (manufactured by Amersham), and used as a probe.

Using the probe, 5 x 10⁵ clones of a human fetal kidney-derived cDNA library (manufactured by Clontech, trade name: Human Fetal Kidney 5'-STRETCH PLUS cDNA Library) were screened by plaque hybridization to obtain 6 independent phage clones (vector: λgt10) which hybridized with the probe (clones 24-1, 40-1, 41A-1, 41B-1, 46-1 and 57-1). All of the hybridization operations were carried out in accordance with the manual attached to the ECL direct nucleic acid labeling detecting system (manufactured by Amersham).

A cDNA fragment in clone 46-1, which is one of the phage clones obtained above, was recovered from the phage vector, and the cDNA fragment was inserted into a plasmid vector.

A 20 µg portion of the phage clone 46-1 DNA was dissolved in 30 µl a buffer containing 10 mM Tris-HCl (pH 7.5), 10 mM magnesium chloride, 100 mM sodium chloride and 1 mM dithiothreitol, and 15 units of *Eco*RI (manufactured by Takara Shuzo) were added to carry out 4 hours of the digestion reaction at 37°C.

The reaction solution was subjected to an agarose electrophoresis to recover a DNA fragment of about 2.3 kb.

The DNA fragment was purified using a QIAEX II gel extraction kit (manufactured by QIAGEN) in accordance with the manual attached.

A 5 µg portion of pBluescript II KS(-) (manufactured by STRATAGENE) was dissolved in 30 µl of a buffer containing 10 mM Tris-HCl (pH 7.5), 10 mM magnesium chloride, 100 mM sodium chloride and 1 mM dithiothreitol, and 15 units of *Eco*RI (manufactured by Takara Shuzo) were added to carry out the digestion reaction at 37°C for 2 hours.

An *Eco*RI-digested DNA fragment was recovered from the reaction solution by ethanol precipitation.

The dephosphorylation reaction of the DNA fragment was carried out at 60°C for 30 minutes using 0.5 unit of alkaline phosphatase (manufactured by Takara Shuzo; derived from *E*. *coli* C75) in 30 µl of a buffer containing 50 mM Tris-HCl (pH 9.0) and 1 mM magnesium chloride.

The reaction solution was subjected to an agarose electrophoresis to recover a DNA fragment of about 3 kb.

The DNA fragment was purified using a QIAEX II gel extraction kit (manufactured by QIAGEN) in accordance with the manual attached.

A 150 ng portion of the EcoRI-digested DNA fragment (about 2.3 kb) obtained from the aforementioned clone 46-1 and 50 ng of the EcoRI-alkaline phosphatase-treated pBluescript II KS(-) fragment were dissolved in 20 ul of a buffer containing 66 mM Tris-HCl (pH 7.5), 6.6 mM magnesium chloride, 10 mM dithiothreitol and 0.1 mM adenosine triphosphate, and 175 units of T4 DNA ligase (manufactured by Takara Shuzo) were added to carry out the ligation reaction at 16°C for 16 hours.

Using the recombinant plasmid DNA obtained by the ligation reaction, an *E*. *coli* DH5α was transformed in the usual way to obtain a plasmid p46-1.

Determination of nucleotide sequence of the cDNA fragment contained in this plasmid revealed that the cDNA of about 2.3 kb described in SEQ ID NO:2 is contained in the plasmid p46-1 and an open reading frame (to be referred to as ORF hereinafter) of 1,425 bp is present in the cDNA.

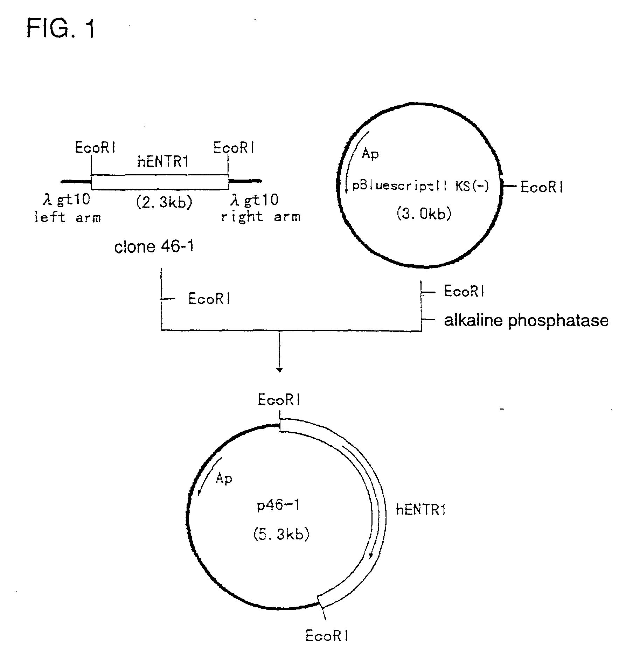

The structure of the p46-1 is shown in Fig. 1.

The novel polypeptide comprised of 475 amino acids described in SEQ ID NO:1 was encoded in the ORF.

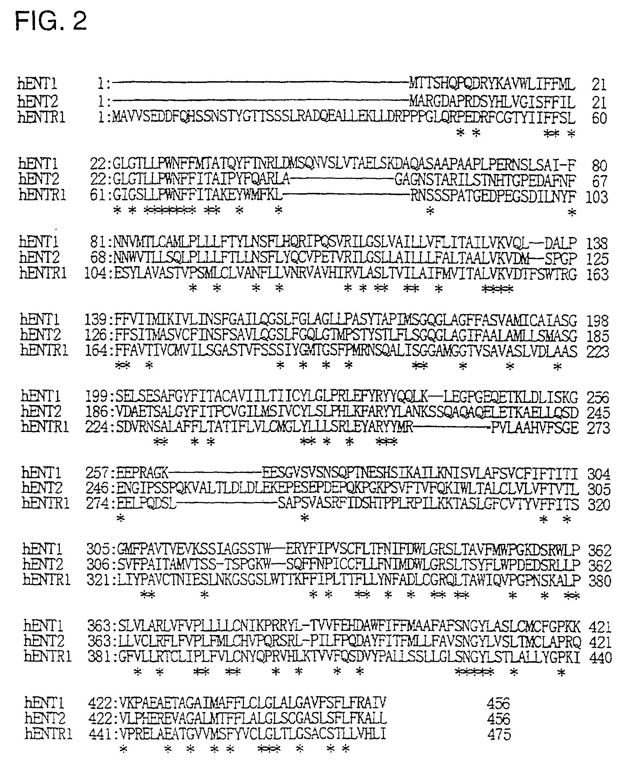

Using an analyzing program {Bestfit included in Wisconsin Package Ver 9.1 (Genetics Computer Group, USA)}, this amino acid sequence was compared with those of the known nucleoside transporters hENT1 and hENT2 (Fig. 2).

The polypeptide and hENT1 exhibited 35% identity at the amino acid sequences and showed 48% of homology when analogous amino acids are taken into consideration.

The polypeptide and hENT2 exhibited 33% identity at the amino acid sequences and showed 43% of homology when analogous amino acids are taken into consideration.

It has been reported that transmembrane regions are important for the substrate specificity and transport activity in various channels and transporters {*FEBS Lett.,* 413, 142 (1997), *J. Biol*. *Chem*., 269, 14865 (1994), *Biochemistry,* 37, 1322 (1998)}.

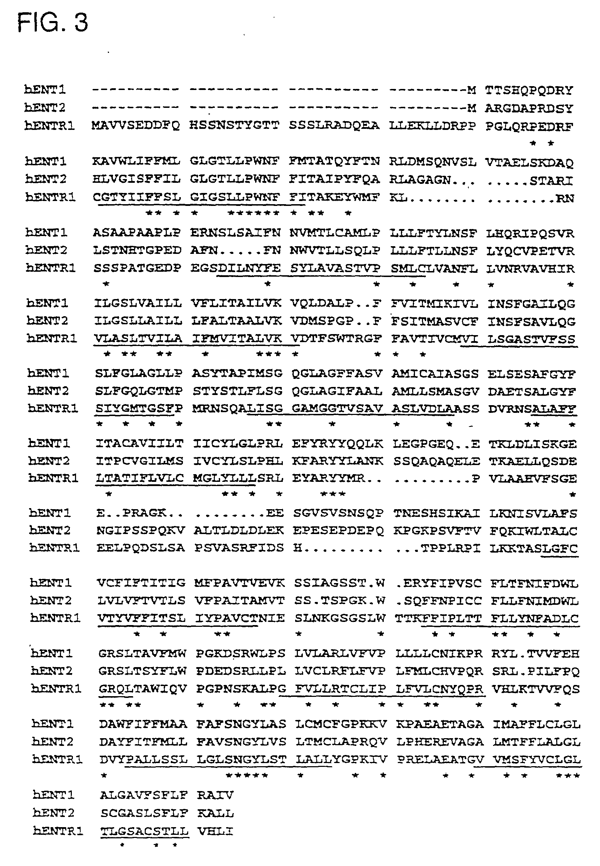

When amino acid sequence of putative transmembrane region of the polypeptide was compared with those of known nucleoside transporters {*Nature Medicine,* 3, 89 (1997)}, they showed high homology in the portions presumed to be transmembrane regions (Fig. 3).

Based on the above results, it is considered that the ORF encodes the novel nucleoside transporter (hENTR1).

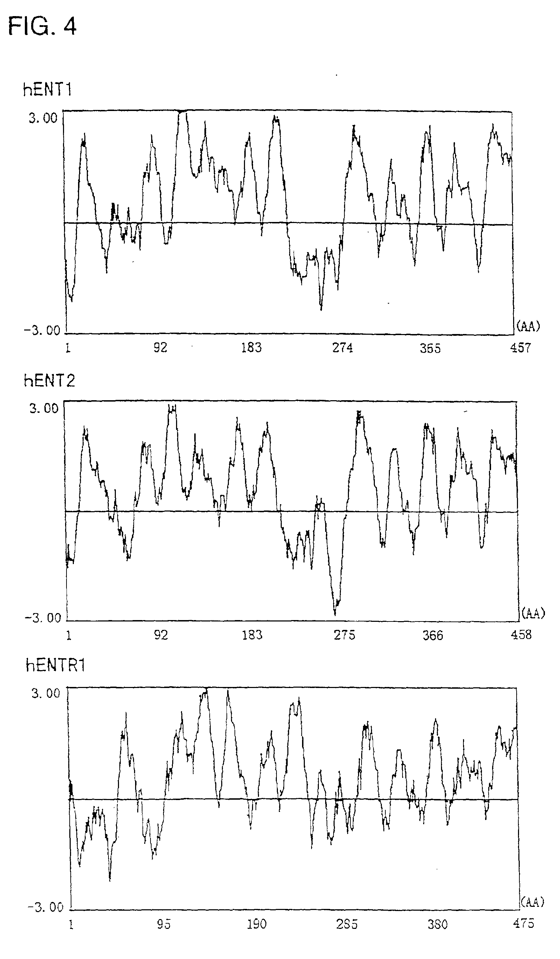

A figure in which hydrophobic plots are compared based on the amino acid sequences of the novel transporter hENTR1 encoded by p46-1 and known nucleoside transporters hENT1 and hENT2 is shown (Fig. 4).

### Example 2 Expression analysis of mRNA by Northern hybridization

A 15 µg portion of the plasmid pHENTlhomologue prepared in Example 1 was dissolved in 50 µl of a buffer containing 10 mM Tris-HCl (pH 7.5), 10 mM magnesium chloride, 100 mM sodium chloride and 1 mM dithiothreitol, and 30 units of EcoRI (manufactured by Takara Shuzo) were added to carry out the digestion reaction at 37°C for 4 hours.

Using the reaction solution, phenol-chloroform extraction and ethanol precipitation were carried out to recover the DNA fragment.

A 1 µg portion of the DNA fragment was dissolved in 50 µl of a buffer containing 40 mM Tris-HCl (pH 8.0), 6 mM magnesium chloride, 2 mM spermidine, 10 mM dithiothreitol, 1 mM ATP, 1 mM CTP, 1 mM GTP, 0.65 mM UTP and 0.35 mM digoxigenin-11-UTP, and 40 units of T7 RNA polymerase (manufactured by Boehringer-Mannheim) were added to carry out the *in vitro* transcription reaction at 37°C for 2 hours.

After the reaction, a digoxigenin-labeled cRNA probe was recovered from the reaction solution by ethanol precipitation.

Using this probe, Northern hybridization was carried out on a poly(A)⁺ RNA filter {filter of Human Multiple Tissue Northern Blots (manufactured by Clontech)} of the human heart, brain, placenta, lung, liver, skeletal muscle, kidney and pancreas under the following conditions.

The filter was soaked in a buffer containing 50% formamide, 5 x SSC (composition of 1 x SSC comprises 150 mM sodium chloride and 15 mM sodium citrate), 0.5% sodium dodecyl sulfate (to be referred to as SDS hereinafter), 2% of a blocking reagent (manufactured by Boehringer-Mannheim) and 0.1 mg/ml salmon sperm DNA (to be referred to as hybridization buffer hereinafter), and the pre-hybridization was carried out at 70°C for 2 hours.

The filter was soaked in the hybridization buffer in which the aforementioned digoxigenin-labeled cRNA probe had been dissolved to a concentration of 1 µg/ml, and the hybridization was carried out at 70°C for 15 hours.

The filter was washed once under a condition of 10 minutes of soaking at 70°C in a buffer of 2 x SSC and 0.1% SDS and then three times under a condition of 30 minutes of soaking at 70°C in a buffer of 0.2 x SSC and 0.1% SDS.

The filter was washed twice under a condition of 15 minutes of soaking at room temperature in a buffer containing 100 mM maleic acid (pH 7.5) and 150 mM sodium chloride (to be referred to as DIG I buffer hereinafter) to remove SDS.

The filter was soaked in a buffer containing 100 mM maleic acid (pH 7.5), 150 mM sodium chloride and 1% of the blocking reagent (to be referred to as DIG II buffer hereinafter) to carry out the blocking at room temperature for 1 hour.

The filter was soaked in a solution prepared by diluting an alkaline phosphatase-labeled anti-digoxigenin antibody Fab fragment (manufactured by Boehringer-Mannheim) 10,000 times with DIG II buffer, and the antigen-antibody reaction was carried out at room temperature for 30 minutes.

The filter was washed three times under a condition of 30 minutes of soaking at room temperature in DIG I buffer to remove excess antibody and then equilibrated by soaking it in a buffer containing 100 mM Tris-HCl (pH 9.0), 100 mM sodium chloride and 50 mM magnesium chloride (to be referred to as DIG III buffer hereinafter) for 5 minutes.

The filter was soaked in a luminous substrate CDP-Star (manufactured by Boehringer-Mannheim) solution prepared by diluting it 100 fold with DIG III, at room temperature for 15 minutes, to effect emission of the signal which was then detected by autoradiography.

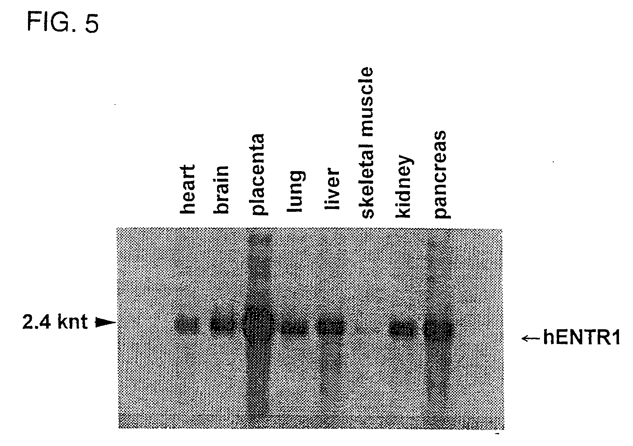

The results are shown in Fig. 5. A band of about 2.4 kilonucleotides was observed in all of the organs of the heart, brain, placenta, lung, liver, skeletal muscle, kidney and pancreas. Particularly strong expression was observed in the placenta and the pancreas.

### Example 3 Cloning of hENT1-related protein (hENTR1) rat counterpart cDNA

Using amino acid regions conserved in hENT1 {*Nature Medicine*, 3, 89 (1997)} and the novel nucleoside transporter hENTR1, the degenerate primers shown in SEQ ID NO:7 and SEQ ID NO:8 were designed and synthesized (n indicates any one of a, c, g and t, y indicates c or t, and r indicates a or g).

PCR was carried out under the following conditions using 50 µl of a reaction solution containing 1.0 µM of each of the thus obtained two primers, 2 µl of a cDNA library prepared from a rat kidney-derived mRNA, a dNTP (dATP, dGTP, dCTP, dTTP) mixture having 200 µM of each component, 2.5 units of Taq Gold (manufactured by Perkin-Elmer) and 1 x Taq Gold buffer (Mg plus).

That is, using TaKaRa PCR Thermal Cycler 480, the reaction solution was heated at 95°C for 10 minutes, subjected to 30 cycles of the reaction using steps of 1 minute at 94°C and 1 minute at 50°C as one cycle and then heated at 72°C for 8 minutes.

A 5 µl portion of the thus obtained PCR reaction solution was applied to an agarose electrophoresis to confirm that the predicted DNA fragment of about 1 kb was amplified, and then the DNA fragment was recovered using QIAEX II Gel Extraction Kit (manufactured by QIAGEN) in accordance with its manual.

Ligation reaction of 50 ng of the thus recovered DNA fragment with 50 ng of pT7Blue(R)T-Vector (manufactured by Novagen) was carried out using DNA ligation kit Ver. 2 (manufactured by Takara Shuzo) in accordance with its manual. Using the recombinant plasmid DNA obtained by the reaction, an *E. coli* JM109 was transformed in the usual way to obtain a plasmid pRENTR1-1kb.

As a result of restriction enzyme analysis, it was revealed that *Bam*HI recognition sequence is present at two positions in the insert DNA fragment contained in the plasmid pRENTR1-1kb and a DNA fragment of about 0.7 kb is cut out from the insert DNA fragment.

Thus, a 30 µg portion of the plasmid pRENTR1-1kb was dissolved in 50 µl of a buffer containing 20 mM Tris-HCl (pH 8.5), 10 mM magnesium chloride, 1 mM dithiothreitol and 100 mM potassium chloride, and 40 units of *Bam*HI (manufactured by Takara Shuzo) were added to carry out 3 hours of the digestion reaction at 37°C. The reaction solution was subjected to an agarose electrophoresis to recover a DNA fragment of the *Bam*HI fragment (0.7 kb) using a QIAEX II Gel Extraction Kit (manufactured by QIAGEN).

The DNA fragment was labeled with horseradish peroxidase using ECL direct nucleic acid labeling detecting system (manufactured by Amersham) and used as a probe.

Using this probe, 3 x 10⁵ clones of a rat kidney-derived cDNA library (manufactured by Clontech, trade name: Rat Kidney 5'-STRETCH PLUS cDNA Library) were screened by plaque hybridization to obtain 6 independent phage clones (vector: λgt11) which hybridized with the probe (clones 3-1, 3-2, 5-1, 6-1, 9-2 and 9-3).

All of the hybridization operations were carried out in accordance with the manual attached to the ECL direct nucleic acid labeling detecting system (manufactured by Amersham).

A cDNA fragment in clone 3-2, which is one of the phage clones obtained above, was recovered from the phage vector, and the cDNA fragment was inserted into a plasmid vector.

A 20 µg portion of phage DNA of the clone was dissolved in 30 µl of a buffer containing 50 mM Tris-HCl (pH 7.5), 10 mM magnesium chloride, 100 mM sodium chloride and 1 mM dithiothreitol, and 15 units of EcoRI (manufactured by Takara Shuzo) were added to carry out 4 hours of the digestion reaction at 37°C. The reaction solution was subjected to an agarose electrophoresis to recover an *EcoRI* fragment (1.7 kb) using a QIAEX II Gel Extraction Kit (manufactured by QIAGEN).

On the other hand, 2 µg of pBluescript II KS(-) (manufactured by STRATAGENE) was dissolved in 30 µl of a buffer containing 50 mM Tris-HCl (pH 7.5), 10 mM magnesium chloride, 100 mM sodium chloride and 1 mM dithiothreitol, and 15 units of EcoRI (manufactured by Takara Shuzo) were added to carry out the digestion reaction at 37°C for 4 hours.

An EcoRI fragment (3.0 kb) was recovered from the reaction solution by ethanol precipitation,

The DNA fragment was dissolved in 30 µl of a buffer containing 50 mM Tris-HCl (pH 9.0) and 1 mM magnesium chloride, and 0.5 unit of alkaline phosphatase (*E. coli* C75) (manufactured by Takara Shuzo) was added to carry out the dephosphorylation reaction at 60°C for 30 minutes.

The reaction solution was subjected to an agarose electrophoresis to recover an EcoRI-alkaline phosphatase-treated fragment (3.0 kb) using a QIAEX II Gel Extraction Kit (manufactured by QIAGEN).

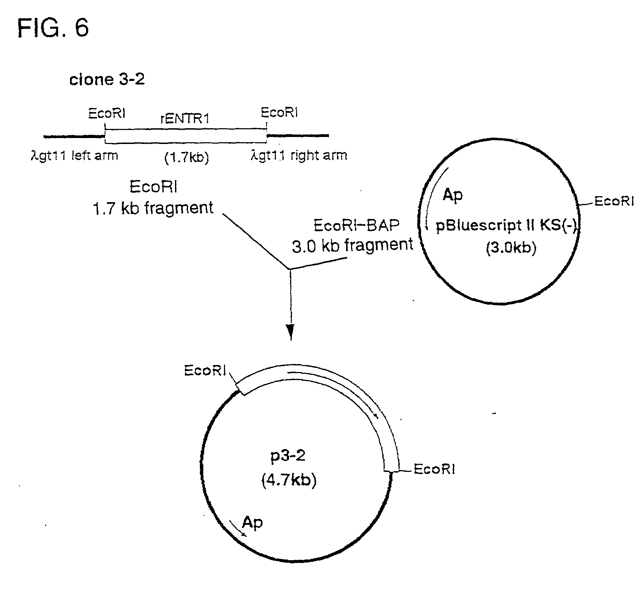

A 50 ng portion of the EcoRI fragment (1.7 kb) derived from the clone 3-2, recovered in the foregoing, and 50 ng of the *Eco*RI-alkaline phosphatase-treated pBluescript II KS(-) fragment (3.0 kb) were subjected to a ligation reaction using DNA ligation kit Ver. 2 (manufactured by Takara Shuzo) in accordance with its manual. Using the recombinant plasmid DNA obtained by this reaction, an E. *coli* JM109 was transformed in the usual way to obtain a plasmid p3-2. Construction steps and a restriction enzyme map of the plasmid p3-2 are shown in Fig. 6.

When nucleotide sequence of the cDNA fragment contained in this plasmid was determined, the cDNA of about 1.7 kb described in SEQ ID NO:6 was contained in the plasmid p3-2, and an open reading frame encoding the novel polypeptide (rENTR1) having the amino acid sequence described in SEQ ID NO:5 was present therein.

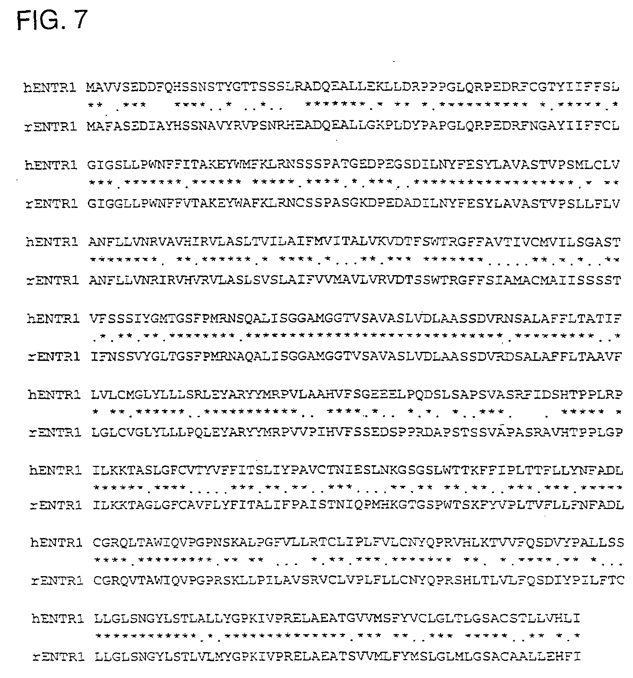

When this amino acid sequence was compared with that of the novel nucleoside transporter (hENTR1) using an analyzing program {GENETYX WIN ver. 2.1 (manufactured by Software)}, 71.8% of identity was found. A result of alignment analysis is shown in Fig. 7.

### Example 4 Expression analysis of mRNA by Northern hybridization

A 10 µg portion of the plasmid pRENTRl-lkb having the nucleotide sequence determined in Example 3 was dissolved in 50 µl of a buffer containing 50 mM Tris-HCl (pH 7.5), 10 mM magnesium chloride, 100 mM sodium chloride and 1 mM dithiothreitol, and 10 units of *Eco*RV (manufactured by Takara Shuzo) was added to carry out the digestion reaction at 37°C for 4 hours. After phenol extraction and ethanol precipitation, the DNA fragment was dissolved in 50 µl of a buffer containing 10 mM Tris-HCl (pH 7.5), 10 mM magnesium chloride and 1 mM dithiothreitol, and 10 units of *Sac*I (manufactured by Takara Shuzo) were added to carry out 4 hours of the digestion reaction at 37°C. The reaction solution was subjected to an agarose electrophoresis to purify an *Eco*RV-*Sac*I fragment (0.4 kb) using a QIAEX II Gel Extraction Kit (manufactured by QIAGEN).

On the other hand, 2 µg of pGEM-7Zf(+) (manufactured by Promega) was dissolved in 50 µl of a buffer containing 33 mM Tris-acetate (pH 7.9), 10 mM magnesium acetate, 0.5 mM dithiothreitol, 66 mM of potassium acetate and 0.01% bovine serum albumin, and 10 units of *Sma*I (manufactured by Takara Shuzo) were added to carry out the digestion reaction at 30°C for 4 hours. After phenol extraction and ethanol precipitation, the DNA fragment was dissolved in 50 µl of a buffer containing 10 mM Tris-HCl (pH 7.5), 10 mM magnesium chloride and 1 mM dithiothreitol, and 10 units of *Sac*I (manufactured by Takara Shuzo) were added to carry out the digestion reaction at 37°C for 4 hours. The reaction solution was subjected to an agarose electrophoresis to purify an *Sma*I-*Sac*I fragment (3.0 kb) using a QIAEX II Gel Extraction Kit (manufactured by QIAGEN).

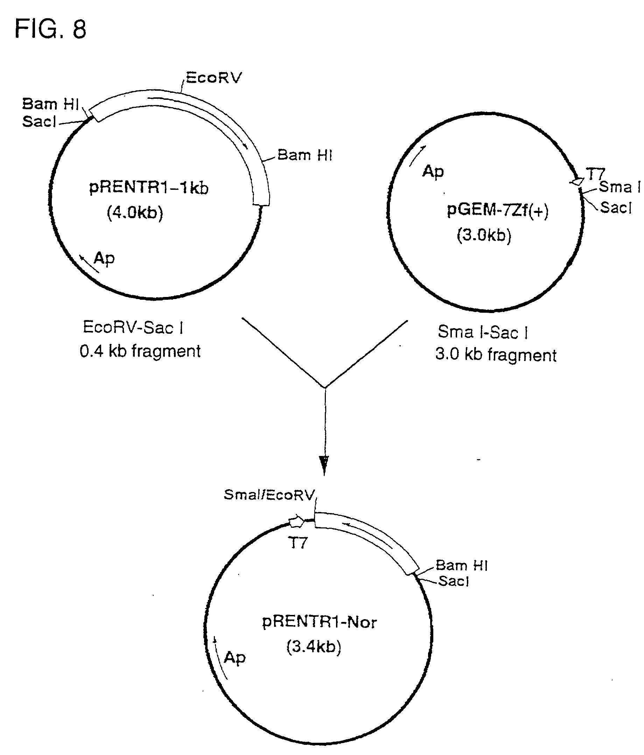

A 50 ng portion of the *Eco*RV-*Sac*I fragment (0.4 kb) derived from the plasmid pRENTR1-1kb, recovered in the foregoing, and 50 ng of the pGEM-7Zf(+) *Sma*I-*Sac*I fragment (3.0 kb) were subjected to a ligation reaction using DNA ligation kit Ver. 2 (manufactured by Takara Shuzo) in accordance with its manual. Using the recombinant plasmid DNA obtained by this reaction, an *E. coli* JM109 was transformed in the usual way to obtain a plasmid pRENTR1-Nor. Construction steps and a restriction enzyme map of the plasmid pRENTR1-Nor are shown in Fig. 8.

A 10 µg portion of the thus prepared plasmid pRENTR1-Nor was dissolved in 50 µl of a buffer containing 20 mM Tris-HCl (pH 8.5), 10 mM magnesium chloride, 1 mM dithiothreitol and 100 mM potassium chloride, and 30 units of BamHI (manufactured by Takara Shuzo) were added to carry out the digestion reaction at 37°C for 3 hours. Using the reaction solution, phenol-chloroform extraction and ethanol precipitation were carried out to recover the DNA fragment.

A 1 µg portion of the DNA fragment was dissolved in 50 µl of a buffer containing 40 mM Tris-HCl (pH 8.0), 6 mM magnesium chloride, 2 mM spermidine, 10 mM dithiothreitol, 1 mM ATP, 1 mM CTP, 1 mM GTP, 0.65 mM UTP and 0.35 mM digoxigenin-11-UTP, and 40 units of T7 RNA polymerase (manufactured by Boehringer-Mannheim) were added to carry out the *in vitro* transcription reaction at 37°C for 2 hours.

A digoxigenin-labeled cRNA probe was recovered from the thus obtained reaction solution by ethanol precipitation.

Using this probe, Northern hybridization was carried out under the following conditions on poly(A)⁺ RNA filters {filter of Rat Multiple Tissue Northern Blots (manufactured by Clontech) and filter of Mouse Multiple Tissue Northern Blots (manufactured by Clontech)} of the heart, brain, spleen, lung, liver, skeletal muscle, kidney and testis derived from rat and mouse, respectively.

The filter was soaked in a hybridization buffer containing 50% of formamide, 5 x SSC (composition of 1 x SSC comprises 150 mM sodium chloride and 15 mM sodium citrate), 0.5% SDS, 2% of a blocking reagent (manufactured by Boehringer-Mannheim) and 0.1 mg/ml salmon sperm DNA, and the pre-hybridization was carried out at 70°C for 2 hours.

The filter was soaked in the hybridization buffer in which the aforementioned digoxigenin-labeled cRNA probe had been dissolved to a concentration of 1 µg/ml, and the hybridization was carried out at 70°C for 15 hours.

The filter was washed once under a condition of 10 minutes of soaking at 70°C in a buffer of 2 x SSC and 0.1% SDS and then three times under a condition of 30 minutes of soaking at 70°C in a buffer of 0.2 x SSC and 0.1% SDS.

The filter was washed twice under a condition of 15 minutes of soaking at room temperature in the DIG I buffer to remove SDS.

The filter was soaked in the DIG II buffer to carry out the blocking at room temperature for 1 hour.

The filter was soaked in a solution prepared by diluting an alkaline phosphatase-labeled anti-digoxigenin antibody Fab fragment (manufactured by Boehringer-Mannheim) 10,000 fold with the DIG II buffer, and the antigen-antibody reaction was carried out at room temperature for 30 minutes.

The filter was washed three times under a condition of 30 minutes of soaking at room temperature in the DIG I buffer to remove excess antibody and then equilibrated by soaking it in the DIG III buffer for 5 minutes.

The filter was soaked in a luminous substrate CDP-Star (manufactured by Boehringer-Mannheim) solution prepared by diluting it 100 fold with the DIG III buffer, at room temperature for 15 minutes, to effect emission of the signal which was then detected by autoradiography.

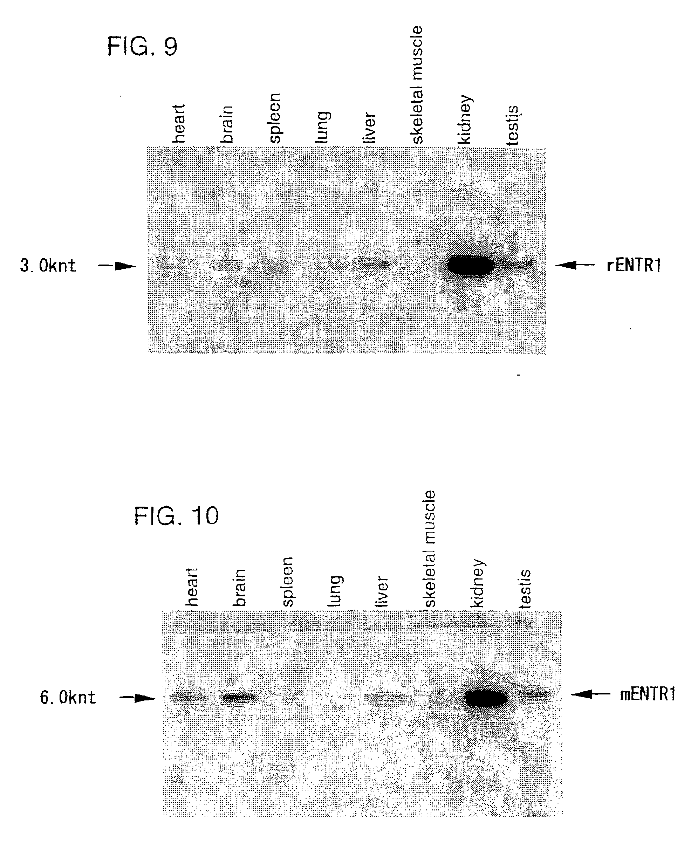

The results for rat are shown in Fig. 9, and the results for mouse in Fig. 10. A band of about 3 kilonucleotides was observed in the case of rat, and a band of about 6 kilonucleotides in the case of mouse. A strong expression was observed in the kidney in both cases.

### Industrial Applicability

The use of a novel transporter polypeptide obtained by the present invention, a DNA encoding this polypeptide and an antibody capable of recognizing the polypeptide makes it possible to provide a preventive agent or a therapeutic agent for ischemic heart disease, cerebral disorder at the time of stroke, immune response accompanied by organ transplantation, malignant tumor, nephritis, pancreatitis or hypertension, an agent for increasing activity of an antiviral agent or a malignant tumor treating agent, an analgesic or an antiplatelet agent or an agent for reducing side effects at the time of chemotherapy, for mammals.

### Sequence Listing Free Text

SEQ ID NO:3 - Description of Artificial Sequence: synthetic DNA
SEQ ID NO:4 - Description of Artificial Sequence: synthetic DNA
SEQ ID NO:7 - Description of Artificial Sequence: synthetic DNA
SEQ ID NO:8 - Description of Artificial Sequence: synthetic DNA

## Claims

1. A polypeptide which comprises the amino acid sequence described in SEQ ID NO:1 or 5.

2. A polypeptide which comprises an amino acid sequence of the amino acid sequence described in SEQ ID NO:1 or 5, wherein one or several amino acids are deleted, substituted or added and has a nucleoside transporting activity.

3. A DNA which encodes the polypeptide of claim 1 or 2.

4. A DNA which has the nucleotide sequence described in SEQ ID NO:2 or 6.

5. A DNA which hybridizes with the DNA of claim 3 or 4 under stringent conditions and encodes a polypeptide having a nucleoside transporting activity.

6. A recombinant DNA which is obtained by inserting the DNA of any one of claims 3 to 5 into a vector.

7. The recombinant DNA according to claim 6, wherein the recombinant DNA is a plasmid p46-1 or p3-2.

8. A transformant which harbours the recombinant DNA of claim 6 or 7.

9. The transformant according to claim 8, wherein the transformant is a transformant selected from a microorganism, an animal cell, a plant cell and an insect cell.

10. The transformant according to claim 9, wherein the microorganism is a microorganism belonging to the genus *Escherichia*.

11. The transformant according to claim 10, wherein the microorganism belonging to the genus *Escherichia* is *Escherichia coli* JM109/p46-1 (FERM BP-6462) or *Escherichia coli* JM109/p3-2 (FERM BP-6830).

12. A method for producing the polypeptide of claim 1 or 2, which comprises culturing the transformant of any one of claims 8 to 11 in a medium to form and accumulate the polypeptide of claim 1 or 2 in the culture, and subsequently recovering the polypeptide from the culture.

13. An oligonucleotide which is selected from oligonucleotides having a sequence identical to continuous 5 to 60 bases in a nucleotide sequence of the DNA of any one of claims 3 to 5, an oligonucleotide having a sequence complementary to said oligonucleotide and oligonucleotide derivatives of these oligonucleotides.

14. The oligonucleotide according to claim 13, which is selected from oligonucleotide derivatives in which a phosphodiester linkage in the oligonucleotide is substituted by a phosphorothioate bond, an oligonucleotide derivative in which phosphodiester linkage in the oligonucleotide is substituted by a N3'-P5' phosphoamidate bond, an oligonucleotide derivative in which a ribose and phosphodiester linkage in the oligonucleotide are substituted by peptide-nucleic acid linkage, an oligonucleotide derivative-in which uracil in the oligonucleotide is substituted by C-5 propynyluracil, an oligonucleotide derivative in which uracil in the oligonucleotide is substituted by C-5 thiazoleuracil, an oligonucleotide derivative in which cytosine in the oligonucleotide is substituted by C-5 propynylcytosine, an oligonucleotide derivative in which cytosine in the oligonucleotide is substituted by phenoxazine-modified cytosine, an oligonucleotide derivative in which ribose in the DNA is substituted by 2'-O-propylribose and an oligonucleotide derivative in which ribose in the oligonucleotide is substituted by 2'-methoxyethoxyribose.

15. A method for detecting mRNA encoding the polypeptide of claim 1 or 2 using the oligonucleotide of claim 13 or 14.

16. A method for inhibiting expression of the polypeptide of claim 1 or 2 using the oligonucleotide of claim 13 or 14.

17. An antibody which recognizes the polypeptide of claim 1 or 2.

18. An immunological detection method of the polypeptide of claim 1 or 2 or an immunohistological staining method, which comprises using the antibody of claim 17.

19. An immunohistological staining agent which comprises the antibody of claim 17.

20. A method for screening a compound capable of changing the activity to transport a nucleoside of the polypeptide of Claim 1 or 2, which comprises contacting said polypeptide with a test sample.

21. A compound obtainable by the method of claim 20.

22. A method for screening a compound capable of changing expression of a gene encoding the polypeptide of claim 1 or 2, which comprises contacting a cell expressing said polypeptide with a test sample.

23. The screening method according to claim 22, wherein the detection of changes in the expression of the gene encoding the polypeptide of claim 1 or 2 is carried out by detecting mRNA encoding said polypeptide using the method of claim 15.

24. The screening method according to claim 22, wherein the detection of changes in the expression of the gene encoding the polypeptide of claim 1 or 2 is carried out by detecting said polypeptide using the method of claim 18.

25. A compound obtainable by any one of the methods of claims 22 to 24.

26. A preventive agent or a therapeutic agent for ischemic heart disease, cerebral disorder at the time of stroke, immune response accompanied by organ transplantation, malignant tumor, nephritis, pancreatitis or hypertension in a mammal, which comprises the polypeptide of claim 1 or 2.

27. An agent for increasing activity of an antiviral agent or a malignant tumor treating agent for mammal, which comprises the polypeptide of claim 1 or 2.

28. An analgesic or an antiplatelet agent for a mammal, which comprises the polypeptide of claim 1 or 2.

29. An agent for reducing side effects at the time of chemotherapy of a mammal, which comprises the polypeptide of claim 1 or 2.

30. A preventive agent or a therapeutic agent for ischemic heart disease, cerebral disorder at the time of stroke, immunoreaction accompanied by organ transplantation, malignant tumor, nephritis, pancreatitis or hypertension in a mammal, which comprises the oligonucleotide of claim 13 or 14.

31. An agent for increasing activity of an antiviral agent or a malignant tumor treating agent for a mammal, which comprises the oligonucleotide of claim 13 or 14.

32. An analgesic or an antiplatelet agent for a mammal, which comprises the oligonucleotide of claim 13 or 14.

33. An agent for reducing side effects at the time of chemotherapy of a mammal, which comprises the oligonucleotide of claim 13 or 14.

34. A preventive agent or a therapeutic agent for ischemic heart disease, cerebral disorder at the time of stroke, immune response accompanied by organ transplantation, malignant tumor, nephritis, pancreatitis or hypertension in a mammal, which comprises the antibody of claim 17.

35. An agent for increasing activity of an antiviral agent or a malignant tumor treating agent for a mammal, which comprises the antibody of claim 17.

36. An analgesic or an antiplatelet agent for a mammal, which comprises the antibody of claim 17.

37. An agent for reducing side effects at the time of chemotherapy of a mammal, which comprises the antibody of claim 17.

38. The preventive agent or a therapeutic agent for ischemic heart disease, cerebral disorder at the time of stroke, immune response accompanied by organ transplantation, malignant tumor, nephritis, pancreatitis or hypertension according to any one of claims 26, 30 and 34, wherein the mammal is human.

39. The agent for increasing activity of an antiviral agent or a malignant tumor treating agent according to any one of claims 27, 31 and 35, wherein the mammal is human.

40. The analgesic or antiplatelet agent according to any one of claims 28, 32 and 36, wherein the mammal is human.

41. The agent for reducing side effects at the time of chemotherapy according to any one of claims 29, 33 and 37, wherein the mammal is human.

42. A promoter DNA which controls transcription of a gene encoding the polypeptide of claim 1 or 2.

43. A method for screening a compound capable of changing efficiency of transcription by the promoter DNA of claim 42, which comprises contacting a transformant harboring a plasmid containing said promoter and a reporter gene connected to the downstream of said promoter DNA with a test sample and measuring the content of translation product of said reporter gene.

44. The screening method according to claim 43, wherein the reporter gene is a gene selected from a chloramphenicol acetyltransferase gene, a β-galactosidase gene, a luciferase gene and a green fluorescent protein gene.

45. A compound obtainable by the method of claim 43 or 44.
